(19) Europäisches Patentamt
European Patent Office
Office européen des brevets

(11) EP 1 392 649 B1

(12) EUROPÄISCHE PATENTSCHRIFT

(45) Veröffentlichungstag und Bekanntmachung des Hinweises auf die Patenterteilung:
**12.04.2006 Patentblatt 2006/15**

(21) Anmeldenummer: **02737878.5**

(22) Anmeldetag: **06.03.2002**

(51) Int Cl.:
***C07C 303/02*** (2006.01) ***C07C 67/08*** (2006.01)
***C07C 309/17*** (2006.01) ***B01F 17/00*** (2006.01)
***C14C 9/00*** (2006.01)

(86) Internationale Anmeldenummer:
**PCT/EP2002/002471**

(87) Internationale Veröffentlichungsnummer:
**WO 2002/085845 (31.10.2002 Gazette 2002/44)**

# (54) MISCHUNGEN VON SULFOGRUPPEN ENTHALTENDEN ESTERN MEHRBASIGER ORGANISCHER SÄUREN MIT LANGKETTIGEN ALKANOLEN

MIXTURES OF SULFOGROUP-CONTAINING ESTERS OF POLYBASIC ORGANIC ACIDS WITH LONG-CHAIN ALKANOLS

MELANGES DE SULFOGROUPES CONTENANT DES ESTERS D'ACIDES ORGANIQUES POLYBASIQUES AVEC DES ALCANOLS A CHAINE LONGUE

(84) Benannte Vertragsstaaten:
**AT BE CH CY DE DK ES FI FR GB GR IE IT LI LU MC NL PT SE TR**

(30) Priorität: **08.03.2001 DE 10111196**

(43) Veröffentlichungstag der Anmeldung:
**03.03.2004 Patentblatt 2004/10**

(73) Patentinhaber: **BASF Aktiengesellschaft
67056 Ludwigshafen (DE)**

(72) Erfinder:
- **LUNKWITZ, Ralph
67434 Neustadt (DE)**
- **SEITZ, Andreas
67134 Birkenheide (DE)**
- **PABST, Gunther
68165 Mannheim (DE)**

(74) Vertreter: **Isenbruck, Günter et al
Isenbruck, Bösl, Hörschler, Wichmann, Huhn
Patentanwälte
Theodor-Heuss-Anlage 12
68165 Mannheim (DE)**

(56) Entgegenhaltungen:
**EP-A- 0 193 832** **DE-A- 1 669 347**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft komplexe Mischungen von Sulfogruppen enthaltenden Estern mehrbasiger organischer Säuren mit langkettigen Alkanolen, Alkoxyalkanolen und Diolen, insbesondere Dickölen aus der Oxosynthese, ihre Herstellung und ihre Verwendung als preisgünstige, gut wirksame Hilfsmittel bei der Lederherstellung und als Emulgatoren.

Bei der Herstellung von Leder werden neben den eigentlichen organischen und/oder anorganischen Gerbstoffen zahlreiche Hilfsmittel eingesetzt, die die Eigenschaften des Leders, insbesondere seine Schmiegsamkeit, seinen Griff und sein Gebrauchsverhalten, insbesondere seine Wasseraufnahme und -durchlässigkeit wesentlich mitbestimmen.

Eine bedeutende Gruppe derartiger Hilfsmittel sind die sogenannten fettenden Ausrüstungsmittel, die, in das Leder eingelagert, die Verhärtung des Leders verhindern und in speziellen Ausführungsformen als Hydrophobiermittel seine Wasserfestigkeit verbessern sollen. Der durch die Fettungs- bzw. Hydrophobiermittel erzielte Effekt soll möglichst dauerhaft sein und auch Behandlungen des ausgerüsteten Leders mit Wasser, wäßrigen Tensidlösungen und ggf. auch Mitteln zur chemischen Reinigung widerstehen.

Aus diesen Forderungen folgt, daß ein Fettungs- bzw. Hydrophobiermittel gut in das Gefüge des Leders eindringen können, es gut abdichten und erweichen und darin wasch- und reinigungsfest verankert werden muß, wenn es seinen Zweck in ausreichendem Maß erfüllen soll. Eine weitere, wesentliche Forderung ist, daß die ausgerüsteten Ledermaterialien keine schmierige Oberfläche aufweisen sollen.

Aus der DE 16 69 347 ist die Fettung von Leder mit wasseremulgierbaren Sulfobernsteinsäure-Estern bekannt. Die so behandelten Leder sind jedoch nicht wasserdicht.
Aus der EP-A-193 832 ist es bekannt, wasserdichte Leder durch eine Behandlung des Leders mit einer Kombination von wasseremulgierbaren Sulfobernsteinsäure-Estern mit imprägnierenden und/oder hydrophobierenden Fettungsmitteln und anschließende Fixierung mit Al-, Cr- oder Zr-Salzen herzustellen.

In der EP-A-372 746 ist für die Fettung von Leder der Einsatz von Copolymeren aus einem überwiegenden Anteil hydrophober und einem untergeordneten Anteil hydrophiler Monomerer und in der EP-A-412 398 für den gleichen Zweck die Verwendung von Carboxylgruppen enthaltenden Copolymerisaten beschrieben worden.

Einem ähnlichen Prinzip folgt das aus der DE-A-41 29 244 bekannte Fettungsverfahren: Hier werden als Wirksubstanz wäßrige Dispersionen Oligomerer eingesetzt, die Carboxylgruppen, Estergruppen und ggf. auch Polyetherketten aufweisen.

In der DE-A-196 44 242 wird der Einsatz von Umsetzungsprodukten von langkettigen aliphatischen Monocarbonsäure, z.B. Stearinsäure, mit niedermolekularen aliphatischen Hydroxy-polycarbonsäuren, z.B. Zitronensäure, und von Umsetzungsprodukten von Fettalkoholen, z.B. Stearylalkohol, mit Pyromellithsäuredianhydrid im Molverhältnis von etwa 1:2 zur Lederfettung empfohlen. Die festen Umsetzungsprodukte werden unter Einsatz von Emulgatoren in cremeartige Pasten überführt. Sie liefern gute Werte der Wasserdurchtrittszeiten, sind aber aufgrund ihrer pastösen Konsistenz umständlich zu applizieren. Ein weiterer Nachteil ist, daß sie aus relativ teuren Ausgangsmaterialien hergestellt werden müssen.

Aus der DE-A-44 05 205 ist die Verwendung von wasserdispergierbaren Partialestern aus mehrbasigen, vorzugsweise drei- oder vierbasigen cyclischen Carbonsäuren mit monofunktionellen Fettalkoholen exakt definierter Kettenlänge zur Lederfettung bekannt. Beispielsweise wird ein Partialester, hergestellt aus Pyromellithsäuredianhydrid und einem gesättigten $C_{18}$-Fettalkohol im Molverhältnis 1:2 eingesetzt.

Als wichtige Eigenschaften dieser Mittel werden einerseits die sichere Fixierung der amphiphilen Substanzen über die freien Carboxylgruppen genannt, andererseits die genaue Einstellung der Penetration in das Leder ohne die Ausbildung einer schmierigen Oberfläche. Um diese Eigenschaften zu erreichen muß bei dieser Methode allerdings das Molekulargewicht der Partialester sehr exakt eingestellt werden, wozu der Einsatz unverhältnismäßig teurer Fettalkohole definierter Kettenlänge und Struktur erforderlich ist. Außerdem wird nach Einsatz dieser Produkte eine Fixierung durch eine Metallsalzbehandlung, insbesondere eine Chromgerbung empfohlen. Die hier eingesetzten Produkte liegen in der Regel als cremeartige bis feste Dispersionen vor, was die Anwendung erschwert.

Die in der Einleitung erwähnte Oxosynthese ist ein technisch in großem Umfang ausgeführter Prozeß zur Herstellung von Aldehyden und Alkoholen. Bei diesem Prozeß wird in Gegenwart geeigneter Katalysatoren an die Doppelbindung von Olefinen Kohlenmonoxid und Wasserstoff angelagert. Der Oxoprozeß eignet sich zur Herstellung eines sehr breiten Spektrums technisch interessanter aliphatischer Substanzen mit Kohlenstoffketten sehr unterschiedlicher Länge. Er ist wegen seiner großen technischen Bedeutung in vielen Richtungen bearbeitet und modifiziert worden. Eine gute Übersicht über die Technologie des Oxoprozesses findet sich beispielsweise bei B. Cornils, in J.Falbe, *New Syntheses with Carbon-Monoxide*, Springer-Verlag, Berlin (1980), *Ullmanns Encyklopädie der technischen Chemie,* 4.Aufl. Bd.7, Seiten 118 ff., und *Winnacker-Küchler*, = Chemische Technologie, 4.Aufl. Bd. 5, Seiten 537 ff..

In einer wichtigen Ausführungsform, bei der phosphinmodifizierte Kobaltkatalysatoren eingesetzt werden, gelingt es, direkt wertvolle langkettige Alkohole in hohen Ausbeuten herzustellen *(Winnacker-Küchler,* = Chemische Technologie, 4.Aufl. Bd. 5, Seiten 537 ff.).

Wie bei jedem technisch durchgeführten Prozeß kommt es auch bei der Oxosynthese aufgrund parallel laufender Nebenreaktionen und Folgereaktionen zur Bildung von Nebenprodukten, die bei einer Ausführungsform des Prozesses, die auf die Herstellung langkettiger Alkohole gerichtet ist, im wesentlichen eine Mischung höhermolekularer Alkohole, Ether, Ester und Diole darstellen.

Diese Mischung, in der Literatur häufig als "Dicköl" bezeichnet, wird bei der Destillation des rohen Oxoprodulctes als Sumpfprodukt erhalten. Dieses Dicköl, - im Folgenden zur eindeutigen Abgrenzung gegen Dicköle anderer Art als "Oxodicköl" bezeichnet - wird zur Zeit nur nach seinem Brennwert bewertet und stellt somit eine Belastung des Oxoprozesses dar.

Es wurde nun überraschend gefunden, daß komplexe Mischungen von amphiphilen Estern mehrbasiger Säuren und höhermolekularer Alkanole, die sich ausgezeichnet als Emulgatoren, insbesondere für Lederfettungsmittel und Hydrophobiermittel eignen, unter Einsatz der Oxodicköle preisgünstig hergestellt werden können.

Die vorliegende Erfindung betrifft somit sowohl die komplexen Mischungen von amphiphilen Estern mehrbasiger Säuren und höhermolekularer Alkanole, die sich ausgezeichnet als Lederfettungs- und Hydrophobiermittel und als Emulgatoren eignen, und vorzugsweise solcher, die als Alköholkomponenten die Bestandteile von Oxodickölen aufweisen, als auch deren Herstellung, Zubereitungen dieser Estermischungen und deren Verwendung als Emulgatoren und als Fettungs- und Hydrophobiermittel bei der Lederherstellung.

In erster Linie betrifft die vorliegende Erfindung Gemische von Estern zwei- oder dreibasiger Carbonsäuren der Formel I

$$[(MOOC)_{a-b}\text{-}R^1\text{-}(COO\text{-})_b]_m \bullet [\text{-}R^2]_n \bullet [\text{-}R^3\text{-}]_p \bullet [OH]_q \qquad (I)$$

worin a und b jeweils die Ziffern 2 oder 3 bedeuten,wobei $b \leq a$ ist,
m eine ganze Zahl von 1 bis 6, vorzugsweise von 1 bis 3, ist,
wobei die Zusammenhänge

$$0 \leq n \leq (b-2)m + 2$$

$$(m-1) \leq p \leq (b-1)m + 1$$

bestehen

$$q = (b-2)m + 2 - n$$

ist,
M für Wasserstoff oder ein Metalläquivalent steht und

- $R^1$ ein zwei- oder dreiwertiger, gesättigter oder ein- oder zweifach ungesättigter aliphatischer oder cycloaliphatischer Kohlenwasserstoffrest mit 2 bis 6C-Atomen ist, wobei mindestens 50 Mol% dieser Reste $R^1$ gesättigt oder höchstens einfach ungesättigt sind und eine Sulfonsäuregruppe aufweisen, oder ein zwei- oder dreiwertiger, eine Sulfonsäuregruppe tragender aromatischer Kohlenwasserstoffrest mit 6 C-Atomen ist,
- $R^2$ überwiegend verzweigtes, gegebenenfalls durch Alkoxy-, Alkylcarbonyloxy- oder Alkoxycarbonyl substituiertes Alkyl mit 9 bis 51, vorzugsweise 9 bis 45 C-Atomen und
- $R^3$ Alkandiyl mit 10 bis 30 C-Atomen bedeutet,

mit von OH-Gruppen freien Ethern und Estern mit 18 bis 45 C-Atomen und gegebenenfalls Alkanolen der Formel $R^2OH$ und Alkandiolen der Formel $R^3(OH)_2$,
wobei, - bezogen auf die Summe SUG der Gewichte der in der Mischung enthaltenen von OH-Gruppen freien Ether, von OH-Gruppen freien Ester, Alkanole, Alkandiole und $\text{-}OR^{2\text{-}}$und $(\text{-}O)_2R^3$-Gruppen, - der Anteil der $\text{-}OR^2$-Gruppen bis zu 85 %, der Anteil der $(\text{-}O)_2R^{3\text{-}}$Gruppen bis zu 16 %, und der Anteil der von OH-Gruppen freien Ether und Ester bis zu 45 % beträgt.

Es ist zu beachten, daß der oben angegebene Anteil der $\text{-}DR^2$-Gruppen und $(\text{-}O)_2R^{3\text{-}}$Gruppen alle derartigen Gruppen umfaßt, unabhängig davon, in welchem Mischungsbestandteil sie enthalten sind, also sowohl die in den Estern der Formeln I und II enthaltenen Gruppen als auch die in gegebenenfalls vorhandenen Alkanolen und Alkandiolen

enthaltenen $-OR^2$-Gruppen und $(-O)_2R^3$-Gruppen einschließt.

Analog gilt für die Bezugsgröße SUG, daß bei ihrer Berechnung ebenfalls das Gewicht aller $-OR^2$-Gruppen und $(-O)_2R^3$-Gruppen, unabhängig davon, in welchem Mischungsbestandteil sie enthalten sind, eingesetzt wird.

In einer bevorzugten Ausführungsform der Erfindung liegt der Anteil der Alkanole der Formel $R^2OH$ und Alkandiole der Formel $R^3(OH)_2$, bezogen auf das Gesamtgewicht der Mischung, unter 5 Gew.%, vorzugsweise unter 2 Gew.-% insbesondere unter 0,5 Gew.-%.

In einer weiteren bevorzugten Ausführungsform der Erfindung beträgt der Anteil der Alkanole der Formel $R^2OH$ und Alkandiole der Formel $R^3(OH)_2$, bezogen auf das Gesamtgewicht der Mischung, bis zu 65 Gew.-%, vorzugsweise bis zu 60 Gew.-%, insbesondere bis zu 55 Gew.-%.

Bevorzugt sind erfindungsgemäße Gemische von Estern zwei- oder dreibasiger Carbonsäuren der Formel I

$$[(MOOC)_{a-b}\text{-}R^1\text{-}(COO\text{-})_b]_m \bullet [\text{-}R^2]_n \bullet [\text{-}R^3\text{-}]_p \bullet [OH]q \qquad (I),$$

worin a, b, m, n, p, q und M jeweils die oben angegebenen Bedeutungen haben und

$R^1$ ein zweiwertiger, gesättigter oder einfach ungesättigter aliphatischer oder cycloaliphatischer Kohlenwasserstoffrest mit 2 bis 6 C-Atomen ist, wobei mindestens 50 Mol% dieser Reste $R^1$ eine Sulfonsäuregruppe aufweisen, oder ein zweiwertiger, eine Sulfonsäuregruppe tragender aromatischer Kohlenwasserstoffrest mit 6 C-Atomen ist.

Besonders bevorzugt sind erfindungsgemäße Estergemische, bei denen

$R^1$ ein zweiwertiger, gesättigter oder einfach ungesättigter aliphatischer Kohlenwasserstoffrest mit 2 bis 6, insbesondere 2 bis 4, C-Atomen ist, wobei mindestens 50 Mol%, vorzugsweise mindestens 75 Mol%, insbesondere mindestens 90 Mol% dieser Reste eine Sulfonsäuregruppe aufweisen.

Von besonderem Vorteil sind dabei solche erfindungsgemäßen Estergemische bei denen mindestens 50 Mol%, vorzugsweise mindestens 75 Mol%, insbesondere mindestens 90 Mol% dieser Reste gesättigt sind und eine Sulfonsäuregruppe aufweisen.

Bevorzugt sind ferner erfindungsgemäße Estergemische, bei denen

$R^2$ die Reste $R^{2A}$, $R^{2B}$, $R^{2C}$, $R^{2D}$ und $R^{2E}$ umfaßt, wobei
$R^{2A}$ Alkyl(1)- und Alkyl(2)-Reste mit 9 bis 15 C-Atomen, mit einem mittleren Molgewicht von MA
$R^{2B}$ 2-Alkyl-elkyl(1)-Reste mit 18 bis 30 C-Atomen, mit einem mittleren Molgewicht von MB
$R^{2c}$ x-Alkyl-alkyl(y)-Reste mit 18 bis 30 C-Atomen, mit einem mittleren Molgewicht von MC
$R^{2D}$ Alkoxyalkyl(1)-Reste mit 18 bis 30 C-Atomen, mit einem mittleren Molgewicht von MD
$R^{2E}$ x-Alkylcarbonyloxy-alkyl(y)-Reste und/oder x-Alkoxycarbonyl-alkyl(y)-Reste mit 27 bis 51 C-Atomen mit einem mittleren Molgewicht von ME bedeutet,
$R^3$ die Reste $R^{3F}$ und $R^{3G}$ umfaßt, wobei
$R^{3F}$ Alkandiyl(1,2) und/oder 2-Alkyl-alkandiyl(1,3) mit 10 bis 16 C-Atomen, mit einem mittleren Molgewicht von MF
$R^{3G}$ Alkyl-alkandiyl(1,3) mit 18 bis 30 C-Atomen ist, mit einem mittleren Molgewicht von MG bedeutet,

mit von OH-Gruppen freien Ethern und Estern mit 18 bis 45 C-Atomen, und gegebenenfalls Alkanolen der Formel $R^2OH$ und Alkandiolen der Formel $R^3(OH)_2$,
wobei, - bezogen auf die Summe SUG der Gewichte der in der Mischung enthaltenen von OH-Gruppen freien Ether und Ester, Alkanole, Alkandiole und $-OR^2$- und $(-O)_2R^{3}$-Gruppen,-
die Gruppen $-OR^{2A}$ einen Anteil von A[%] = 0 bis 20 Gew.-%,
die Gruppen $-OR^{2D}$ einen Anteil von B[%] = 0 bis 40 Gew.-%,
die Gruppen $-OR^{2C}$ einen Anteil von C[%] = 0 bis 10 Gew.-%,
die Gruppen $-OR^{2D}$ einen Anteil von D[%] = 0 bis 20 Gew.-%,
die Gruppen $-OR^{2E}$ einen Anteil von E[%] = 0 bis 25 Gew.-%,
die Gruppen $(-O)_2R^{3F}$- einen Anteil von F[%] = 0 bis 7,5 Gew.-%,
die Gruppen $(-O)_2R^{3G}$- einen Anteil von G[%] = 0 bis 8 Gew.-%,
und die von OH-Gruppen freien Ether und Ester einen Anteil von H[%] = 0 bis 45 Gew.-% haben.

Die Werte von x und y ergeben sich aus den weiter unten beschriebenen Strukturtypen für die Bestandteile der Reste $-R^2$ und $-R^3$. Vorzugsweise liegt der Zahlenwert von x im Bereich von 9 bis 15, der von y im Bereich von 10 bis 16.

Bevorzugt sind solche erfindungsgemäßen Estergemische, bei denen die Anteile der in den Baugruppen $-OR^2$- und $-OR^3$-enthaltenen Strukturen $-OR^{2A}$, $-OR^{2B}$, $-OR^{2C}$, $-OR^{2D}$, $-OR^{2E}$, $-OR^{3F}O$- und $-OR^{3G}O$- und der Anteil der Ether und Ester im Rahmen der oben angegebenen Grenzen so gewählt werden, daß die durch die Gleichung (GL1)

$$OHZ = 561 \cdot \left( \frac{A[\%]}{MA} + \frac{B[\%]}{MB} + \frac{C[\%]}{MC} + \frac{D[\%]}{MD} + \frac{E[\%]}{ME} \right) + 1120 \cdot \left( \frac{F[\%]}{MF} + \frac{G[\%]}{MG} \right)$$

(GL1)

worin A[%], B[%], C[%], D[%] E[%], F[%] und G[%] die oben genannten prozentualen Anteile der in den Baugruppen $-OR^2$ und $-OR^3$ enthaltenen Strukturen, bezogen auf das Summengewicht SUG, und MA, MB, MC, MD, ME, MF und MG die Molekulargewichte der besagten Strukturen sind, definierte Größe OHZ im Bereich von 65 bis 160, vorzugsweise im Bereich von 90 bis 140, insbesondere von 90 bis 120 liegt.

Als Molekulargewichte sind die sich aus den Formeln der Reste $-OR^2$ und $-OR^3$ ergebenden Zahlenmittel der Molekulargewichte einzusetzen. Die prozentualen Anteile A[%], B[%], C[%], D[%], E[%], F[%],und G[%] der Mischungsbestandteile müssen sich zu (100-H)[%] ergänzen.

Weiterhin sind solche erfindungsgemäßen Mischungen von Estern der Formeln I und II bevorzugt, in denen

$R^1$ Ethan-1,2-diyl, Sulfo-ethan-1,2-diyl, Ethen-1,2-diyl, Propan-1,2-diyl, Propan 1,3-diyl, Sulfopropan-1,2-diyl, Sulfopropan 1,3-diyl, Propen-1,2-diyl, Cyclohexan-1,2- diyl, Sulfocyclohexan-1,2-diyl, Disulfocyclohexan-1,2-diyl, Cyclohex-1-,2-,3-oder 4-en-1,2-diyl, Cyclohex-1,3-, 1,4-, 2,4- oder 2,5-dien-1,2-diyl, Cyclohexan-1,2,4-triyl, Sulfocyclohexan-1,2,4-triyl, Disulfocyclohexan-1,2,4-triyl, Cyclohex-1-,2-,3- oder 4-en-1,2,4-triyl, Cyclohex-1,3-, 1,4-, 2,4- oder 2,5-dien-1,2,4-triyl, Phenylen-1,2, 3- oder 4-Sulfophenylen-1,2 und Phenyl-1,2,4-triyl bedeutet und insbesondere solche in denen

$R^1$ für Ethen-1,2-diyl, Sulfo-ethan-1,2-diyl, Phenylen-1,2 oder 3- oder 4-Sulfophenylen-1,2 steht.

Reste $R^1$, die Sulfongruppen tragen, sind besonders bevorzugt.

Die erfindungsgemäßen Estergemische können auch Komponenten enthalten, die sich bezüglich der Säurereste $R^1$ voneinander unterscheiden.

Bevorzugt sind auch solche erfindungsgemäßen Mischungen von Estern der Formeln I und II, in denen die Zusammensetzung von $R^2$ und $R^3$ dadurch definiert ist, daß

A[%] einen Wert von 4 bis 15, insbesondere von 5 bis 10,
B[%] einen Wert von 20 bis 35, insbesondere von 25 bis 31,
C[%] einen Wert von 1 bis 5, insbesondere von 2 bis 4,
D[%] einen Wert von 3 bis 20, insbesondere von 5 bis 15,
E[%] einen Wert von 5 bis 25, insbesondere von 10 bis 20,
F[%] einen Wert von 0,8 bis 5, insbesondere von 1 bis 4,
G[%] einen Wert von 1 bis 5, insbesondere von 2 bis 4, und
H[%] einen Wert von 10 bis 40, insbesondere von 20 bis 35 hat.

Bei den folgenden näheren Angaben zur Struktur der Reste $-R^2$ und $-R^3$ bedeuten R und R' gleiche oder verschiedene, vorzugsweise lineare, Alkylreste mit 7 bis 13 C-Atomen.

Die Reste $R^{2A}$ sind vorzugsweise lineare Alkyl(1) oder 2-Methyl-alkyl(1)-Reste, die sich von Alkanolen bzw. Methyl-alkanolen (Oxoalkoholen) vom Strukturtyp $R-CH_2CH_2-OH$ bzw. $R-CH(CH_3)-OH$ ableiten, wobei in der Regel der Anteil der unverzweigten Alkanole überwiegt.

Die 2-Alkyl-alkyl(1)-Reste $-R^{2B}$ leiten sich vorzugsweise von verzweigten Alkanolen $HO-R^{2B}$ vom Strukturtyp $R-CH_2-CH_2-CH(CH_2OH)-R'$ ab, die sich aus Aldehyden der Formeln $R-CH_2-CHO$ und $R'-CH_2-CHO$ durch Aldolkondensation, und anschließende Wasserabspaltung und Hydrierung bilden können.

Die x-Alkyl-alkyl(y)-Reste $-R^{2C}$ leiten sich von sekundären Alkanolen $HO-R^{2C}$ vom Strukturtyp $R-CH_2-CH(OH)-CH(CH_3)-R'$ ab. Diese werden wahrscheinlich durch Aldolkondensation aus Aldehyden der Formeln $R-CH_2-CHO$ und $R'-CH_2-CHO$ und anschließende Hydrierung der Aldehydgruppe zur Methylgruppe gebildet.

Die Alkoxyalkylreste $-R^{2D}$ leiten sich von Alkanolen $HO-R^{2D}$ der Strukturtypen $R-CH_2-CH(CH_2OH)-OCH_2-R'$ und $R-CH(CH_2OH)CH_2-OCH_2-R'$ ab. Diese können sich durch Acetalisierung von $R-CH_2-CHO$ mit 2 Mol $R'-CH_2-CH_2OH$, Abspaltung von einem Mol des Alkanols unter Bildung von Vinylethern der Formel II

$$R-CH=CH-O-CH_2-CH_2-R' \qquad (II),$$

und Hydroformylierung der Vinylether bilden.

Die Estergruppen tragenden Reste -$R^{2E}$ leiten sich von einem oder mehreren Alkanolen HO-$R^{2E}$ der Strukturtypen
R-$CH_2$-CH(OH)-CH($CH_2$-OCO-$CH_2$-R)-R',
R-$CH_2$-CH(OH)-CH($CH_2$-OCO-$CH_2$-R')-R',
R-$CH_2$-CH(OH)-CH(COO-$C_2H_4$-R)-R'
R-$CH_2$-CH(OH)-CH(COO-$C_2H_4$-R')-R'
R-$CH_2$-CH(OCO-$CH_2$-R)-CH($CH_2$OH)-R' und
R-$CH_2$-CH(OCO-$CH_2$-R')-CH($CH_2$OH)-R'
ab. Diese können gebildet werden durch eine Cannizzaro-Reaktion (Disproportionierung) von 1 Mol eines der oben genannten Aldole mit einem Mol eines Aldehyds R-$CH_2$-CHO oder R'-$CH_2$-CHO und anschließende Veresterung der dabei entstandenen Carbonsäuren und Alkanole.

Die Alkandiyl-Reste -$R^{3F}$ leiten sich von Diolen $(HO)_2$-$R^{3F}$ der Strukturtypen R-$CH_2$-CH($CH_2$OH)-OH und R-CH($CH_2$OH)-$CH_2$OH ab. Diese können gebildet werden durch Abspaltung eines Mols Olefin aus den Alkoxygruppen der oben genannten Alkoxyalkanole.

Alkyl-alkandiyl-Reste -$R^{3G}$ leiten sich von Diolen $(HO)_2$-$R^{3G}$ des Strukturtyps R-$CH_2$-CH(OH)-CH($CH_2$OH)-R', die durch Hydrierung der oben genannten Aldole erhalten werden können.

Die von OH-Gruppen freien Ether entsprechen der Struktur R-$CH_2CH_2$-O-$CH_2$-R'. Sie werden bei der Hydrierung der Vinylether der Formel II erhalten.

Die von OH-Gruppen freien Ester können linear oder verzweigt sein. Die linearen Ester entsprechen dem Strukturtyp R-$CH_2CH_2$-OCO-$CH_2$R', und bilden sich durch Cannizzaro Disproportionierung aus Aldehyden der Formel R-$CH_2$-CHO und R'-$CH_2$-CHO und anschließende Veresterung der entstandenen Carbonsäuren und Alkanole.

Die verzweigten Ester entsprechen den Strukturtypen
R-$CH_2$-$CH_2$-CH($CH_2$-OCO-$CH_2$-R)-R',
R-$CH_2$-$CH_2$-CH($CH_2$-OCO-$CH_2$-R')-R',
R-$CH_2$-$CH_2$-CH(COO-$C_2H_4$-R)-R' und
R-$CH_2$-$CH_2$-CH(COO-$C_2H_4$-R')-R'
R-$CH_2$-CH(OCO-$CH_2$-R)-CH($CH_3$)-R' und
R-$CH_2$-CH(OCO-$CH_2$-R')-CH($CH_3$)-R'.

Sie können gebildet werden durch Wasserabspaltung und anschließende Hydrierung der oben genannten Estergruppen tragenden Alkanole.

Ein weiterer Gegenstand der vorliegenden Erfindung ist ein Verfahren zur Herstellung der oben beschriebenen Gemische von Estern der Formel I durch Umsetzung funktioneller Derivate zwei- oder dreibasiger Carbonsäuren mit Alkanolen mittlerer und/oder größerer Kettenlänge, das dadurch gekennzeichnet ist, daß man

A) funktionelle Derivate von Di- oder Tricarbonsäuren der Formel III

$$R^1(COX)_a \qquad (III)$$

worin a die Ziffern 2 oder 3 bedeutet,

$R^1$ ein zwei- oder dreiwertiger, gesättigter aliphatischer oder cycloaliphatischer Kohlenwasserstoffrest mit 2 bis 6 C-Atomen ist, der durch eine Sulfonsäuregruppe substituiert ist, oder ein ein- oder zweifach ungesättigter aliphatischer oder cycloaliphatischer Kohlenwasserstoffrest mit 2 bis 6 C-Atomen ist, der durch eine Sulfonsäuregruppe substituiert sein kann,
oder ein zwei- oder dreiwertiger, eine Sulfonsäuregruppe tragender aromatischer Kohlenwasserstoffrest mit 6 C-Atomen ist,
wobei $R^1$ zumindest entweder eine Sulfonsäuregruppe oder eine olefinische Doppelbindung enthält,

X Hydroxy, Niederalkoxy, oder Halogen bedeuten oder zwei X gemeinsam für Sauerstoff stehen,

bei einer Temperatur von ca. 50 bis 180°C, vorzugsweise von 75 bis 150°C, im Molverhältnis von 1:2 bis 1:5, vorzugsweise in Gegenwart von Veresterungs- oder Umesterungskatalysatoren und/oder gegebenenfalls Säurefängern, mit einer Mischung von Alkoholen der Formel $R^2$OH, worin

$R^2$ überwiegend verzweigtes, gegebenenfalls durch Alkoxy-, Alkylcarbonyloxy- oder Alkoxycarbonyl substituiertes Alkyl mit 9 bis 51, vorzugsweise 9 bis 45 C-Atomen bedeutet,

Alkandiolen der Formel $(HO)_2R^3$, worin

$R^3$ Alkandiyl mit 10 bis 30 C-Atomen bedeutet,

und von OH-Gruppen freien Ethern und Estern mit 18 bis 45 C-Atomen umsetzt, und,

B) falls für den Herstellungsschritt A) ein von Sulfonsäuregruppen freies, jedoch mindestens eine olefinische Doppelbindung aufweisendes Carbonsäure-Derivat (III) eingesetzt wird, das gemäß Abschnitt A) erhaltene Ester-Gemisch, gegebenenfalls nach zumindest teilweiser Neutralisation saurer Gruppen, in an sich bekannter Weise mit einem wasserlöslichen Sulfit, Hydrogensulfit oder Disulfit unter Addition des Sulfits bzw. Hydrogensulfits an die Doppelbindung der Gruppe $R^1$ unter Bildung der gewünschten Sulfonsäure umsetzt.

Für X stehendes Halogen ist vorzugsweise Chlor oder Brom. Besonders bevorzugt ist Chlor. Für X stehende Niederalkoxygruppen haben zweckmäßigerweise 1 bis 4, vorzugsweise 1 oder 2 C-Atome.
Die Mengen der im Herstellungsschritt A) miteinander umzusetzenden Reaktanden richten sich nach dem Molgewicht der Carbonsäure-Derivate der Formel III und dem Molgewicht der Mischung der Alkohole $HOR^2$ und Diole $(HO)_2R^3$, das sich in bekannten Weise aus deren OH-Zahl berechnen läßt.
Die Bedingungen der Esterherstellung aus Carbonsäurederivaten und Alkanolen sind an sich bekannt. Die Herstellung der erfindungsgemäßen Estergemische kann daher im Prinzip im Rahmen der bekannten Methoden ausgeführt werden. So kann die Umsetzung der Di- oder Tricarbonsäurederivate der Formel III mit den Mischungen OH-Gruppen enthaltender Verbindungen mit oder ohne Lösungs- oder Verdünnungsmittel erfolgen. Sofern die Verbindungen der Formel III mit der Alkohol- und Diol-Mischung in einem Molverhältnis unter 0,5 umgesetzt werden - d.h. daß die Alkohol- und Diol-Komponenten im Überschuß vorliegen -kann dieser als Lösung- und Verdünnungsmittel für die Reaktion dienen.
Die Esterbildung aus Carbonsäuren, Carbonsäureanhydriden oder Carbonsäure-Niederalkylestern wird zweckmäßigerweise durch Zusatz geringer Mengen saurer Katalysatoren, vorzugsweise von Brønsted-Säuren, beispielsweise anorganischer Säuren wie Schwefel- oder Salzsäure, oder ausreichend starker organischer Säuren wie Ameisensäure oder Benzol- oder Toluolsulfonsäure, katalysiert.
Sofern als funktionelles Säurederivat der Formel III ein Säurehalogenid eingesetzt wird, ist es zweckmäßig, den entstehenden Halogenwasserstoff durch Zusatz einer Base, wie z.B Natrium- Kalium- oder Kalziumcarbonat oder einem organischen Amin zu binden.
Beim Einsatz von Carbonsäuren, Carbonsäureanhydriden, oder Carbonsäure-Niederalkylestem als funktionelle Derivate der Formel III entstehen Wasser bzw. niedere Alkanole. Diese Produkte werden zweckmäßigerweise aus dem Reaktionssystem entfernt, um das Reaktionsgleichgewicht möglichst weit in Richtung auf die gewünschten Estergemische zu verschieben. Dies kann dadurch erfolgen, daß diese leicht flüchtigen Produkte aus dem Ansatz abdestilliert werden, was durch Arbeiten im Vakuum oder durch Zusatz eines inerten Schleppmittels erheblich erleichtert und beschleunigt werden kann.
In der Regel wird beim Herstellungsschritt A) unter den angegebenen Reaktionsbedingungen ein ausreichender Umsatz der Reaktanten innerhalb von 2 bis 5 Stunden erreicht.
Das erfindungsgemäße Verfahren kann unter Verwendung eines einzelnen oder mehrerer verschiedener funktioneller Carbonsäurederivate der Formel III ausgeführt werden.
Bevorzugte erfindungsgemäße Ester-Mischungen werden erhalten, wenn funktionelle Carbonsäurederivate der Formel III eingesetzt werden, in denen

$R^1$ ein zweiwertiger, gesättigter aliphatischer Kohlenwasserstoffrest mit 2 bis 4 C-Atomen, der durch eine Sulfonsäuregruppe substituiert sein kann,
oder ein zweiwertiger, einfach ungesättigter aliphatischer Kohlenwasserstoffrest mit 2 bis 4 C-Atomen,
oder ein zweiwertiger oder dreiwertiger, gesättigter cycloaliphatischer Kohlenwasserstoffrest mit 6 C-Atomen, der durch eine Sulfonsäuregruppe substituiert sein kann
oder ein zwei oder dreiwertiger cycloaliphatischer Kohlenwasserstoffrest mit 6 C-Atomen, der eine oder ggf. zwei Doppelbindungen aufweist,
oder ein zwei oder dreiwertiger, eine Sulfogruppe tragender aromatischer Kohlenwasserstoffrest mit 6 C-Atomen bedeutet.

Insbesondere eignen sich zur Herstellung bevorzugter erfindungsgemäßer Ester-Mischungen funktionelle Carbonsäurederivate der Formel III, in denen

$R^1$ Ethan-1,2-diyl, Sulfo-ethan-1,2-diyl, Ethen-1,2-diyl, Propan-1,2-diyl, Propan 1,3-diyl, Sulfopropan-1,2-diyl, Sulfopropan 1,3-diyl, Propen-1,2-diyl, Cyclohexan-1,2-diyl, Sulfocyclohexan-1,2-diyl, Disulfocyclohexan-1,2-diyl, Cyclohex-1-,2-,3-oder 4-en-1,2-diyl, Cyclohex-1,3-, 1,4-, 2,4- oder 2,5-dien-1,2-diyl, Cyclohexan-1,2,4-triyl, Sulfocyclohexan-1,2,4-triyl, Disulfocyclohexan-1,2,4-triyl, Cyclohex-1-,2-,3- oder 4-en-1,2,4-triyl, Cyclohex-1,3-, 1,4-,

2,4- oder 2,5-dien-1,2,4-triyl, Phenylen-1,2, 3- oder 4-Sulfophenylen-1,2 und Phenyl-1,2,4-triyl bedeutet und insbesondere solche in denen

$R^1$ für Ethen-1,2-diyl, Sulfo-ethan-1,2-diyl, oder 4-Sulfophenylen-1,2.

Als Ausgangsmaterialien der Formel IV kommen beispielsweise in Betracht funktionelle Derivate der Bernsteinsäure, Sulfo-bernsteinsäure Maleinsäure, Glutarsäure, Sulfoglutarsäure, Methylbernsteinsäure, Sulfo-methylbernsteinsäure Citraconsäure, Sulfohexahydrophthalsäure (Sulfo-cyclohexandicarbonsäure), alle isomeren Tetrahydrophthalsäuren, alle isomeren Dihydrophthalsäuren, und Sulfo-phthalsäure, wovon funktionelle Derivate der Maleinsäure und Sulfo-phthalsäure besonders bevorzugt sind.

Vorzugsweise werden bei dem erfindungsgemäßen Verfahren Mischungen von Alkoholen $R^2OH$ und Diolen $R^3(OH)_2$ eingesetzt, die

0 bis 20 Gew.-%, vorzugsweise 4 bis 15 Gew.-% Alkanole $C_9$ - $C_{15}$
0 bis 40 Gew.-%, vorzugsweise 20 bis 35 Gew.-% 2-Alkylalkohole $C_{18}$ - $C_{30}$
0 bis 10 Gew.-%, vorzugsweise 1 bis 5 Gew.-% Sek. Alkohole $C_{18}$ - $C_{30}$
0 bis 20 Gew.-%, vorzugsweise 3 bis 20 Gew.-% Etheralkohole $C_{18}$ - $C_{30}$
0 bis 25 Gew.-%, vorzugsweise 5 bis 25 Gew.-% Esteralkohole $C_{27}$ - $C_{51}$
0 bis 7,5 Gew.-%, vorzugsweise 0,8 bis 5 Gew.-% 1,2-Diole $C_{10}$ - $C_{16}$
0 bis 8 Gew.-%, vorzugsweise 1 bis 5 Gew.-% Diole $C_{18}$ - $C_{30}$
0 bis 45 Gew.-%, vorzugsweise 10 bis 40 Gew.-% von OH-Gruppen-freie Ether $C_{18}$ - $C_{45}$ und Ester $C_{18}$ - $C_{45}$ umfassen.

Die genannten Mischungsbestandteile $R^2OH$ und $R^3(OH)_2$ können selbst Mischungen von Verbindungen verschiedener Strukturtypen darstellen, die sich aus der folgenden Tabelle 1 ergeben.

**Tabelle 1**

| Bezeichnung | Struktur-Typ | Anteilsbereich [Gew.-%] | Bevorzugter Anteilsbereich [Gew.-%] |
|---|---|---|---|
| Oxoalkohole $C_9$ - $C_{15}$ | $R-CH_2-OH$ bzw. $R-CH(CH_3)-OH$ | 0 bis 20 | 4 bis 15 |
| 2-Alkylalkohole $C_{18}$ - $C_{30}$ | $R-CH_2-CH_2-CH(CH_2OH)-R'$ | 0 bis 40 | 20 bis 35 |
| Sek. Alkohole $C_{18}$ - $C_{30}$ | $R-CH_2-CH(OH)-CH(CH_3)-R'$ | 0 bis 10 | 1 bis 5 |
| Etheralkohole $C_{18}$ - $C_{30}$ | $R-CH_2-CH(CH_2OH)-OCH_2-R'$ und $R-CH(CH_2OH)CH_2-OCH_2-R'$ | 0 bis 20 | 3 bis 20 |
| Esteralkohole $C_{27}$ - $C_{51}$ | $R-CH_2-CH(OH)-CH(CH_2-OCO-CH_2-R)-R'$, $R-CH_2-CH(OH)-CH(CH_2-OCO-CH_2-R')-R'$, $R-CH_2-CH(OH)-CH(COO-C_2H_4-R)-R'$ $R-CH_2-CH(OH)-CH(COO-C_2H_4-R')-R'$ $R-CH_2-CH(OCO-CH_2-R)-CH(CH_2OH)-R'$ $R-CH_2-CH(OCO-CH_2-R')-CH(CH_2OH)-R'$ | 0 bis 25 | 5 bis 25 |
| 1,2- und 1,3-Diole $C_{10}$ - $C_{16}$ | $R-CH_2-CH(CH_2OH)-OH$ und $R-CH(CH_2OH)-CH_2OH$ | 0 bis 7,5 | 0,8 bis 5 |
| Alkyl-1,3-Diole $C_{18}$ - $C_{30}$ | $R-CH_2-CH(OH)-CH(CH_2OH)-R'$ | 0 bis 8 | 1 bis 5 |

Tabelle fortgesetzt

| Bezeichnung | Struktur-Typ | Anteilsbereich [Gew.-%] | Bevorzugter Anteilsbereich [Gew.-%] |
|---|---|---|---|
| Ether $C_{18}$ - $C_{45}$ (OH-Gruppen-frei) | R-$CH_2CH_2$-O-$CH_2$-R' | 0 bis 45 | 10 bis 40 |
| Ester $C_{18}$ - $C_{45}$ (OH-Gruppen-frei) | R-$CH_2CH_2$-O-$CH_2$-R' R-$CH_2$-$CH_2$-CH($CH_2$-OCO-$CH_2$-R)-R', R-$CH_2$-$CH_2$-CH($CH_2$-OCO-$CH_2$-R')-R', R-$CH_2$-$CH_2$-CH(COO-$C_2H_4$-R)-R', R-$CH_2$-$CH_2$-CH(COO-$C_2H_4$-R')-R', R-$CH_2$-CH (OCO-$CH_2$-R)-CH ($CH_3$)-R', R-$CH_2$-CH (OCO-$CH_2$-R')-CH ($CH_3$)-R', R-$CH_2$-CH (OCO-$CH_2$-R)-CH($CH_3$)-R, ' R-$CH_2$-CH(OCO-$CH_2$-R')-CH($CH_3$)-R' | | |

Die für das Verfahren einzusetzenden Mischungen der angegebenen Zusammensetzung können dadurch erhalten werden, daß man eine aus der angegebenen Substanzgruppe getroffene Auswahl miteinander mischt, wobei die Mengen so bemessen werden, daß die oben angegebenen Obergrenzen der Anteile der Mischungsbestandteile nicht überschritten werden und die Summe der %-Anteile der gemischten Komponenten 100% beträgt.

Vorzugsweise wählt man die Komponenten und ihre Anteile in der Mischung so aus, daß die Mischung eine OH-Zahl zwischen 65 und 160, insbesondere zwischen 90 und 140 mg KOH / g aufweist.

Wird beim erfindungsgemäßen Verfahren das funktionelle Derivat III mit dem Alkanol- und Diol-Gemisch in Molverhältnissen von etwa 1:2 umgesetzt, so werden spezielle erfindungsgemäße Ester-Gemische erhalten, die in der Regel weniger als 5 Gew.-%, vorzugsweise weniger als 2 Gew.-%, insbesondere weniger als 0,5 Gew.-% Alkanole und Diole enthalten.

Wird das funktionelle Derivat III mit dem Alkanol- und Diol-Gemisch in Molverhältnissen unter 0,5, z.B. von 1: 2,5 bis 1:5, umgesetzt, so werden spezielle Mischungen erhalten, die höhere Anteile an Alkanolen und Diolen , z.B. ca. 37 Gew.-% bzw. 55 Gew.-%, aufweisen, die sich besonders gut zu anwendungsfertigen Lederfettungs- und -hydrophobierungs-Zubereitungen verarbeiten lassen, weil sie leicht mit weiteren Fettungs- und Hydrophobierungsmitteln und Hilfsmitteln gemischt werden können und weil auch die überschüssige Alkanol- und Diol-Komponenten eine gute fettende Wirkung zeigen.

Ein ganz besonderer Vorteil der vorliegenden Erfindung besteht darin, daß die erfindungsgemäßen Estergemische auf sehr einfache Weise durch Umsetzung der funktionellen Carbonsäurederivate der Formel III mit sogenannten Oxo-Dickölen als Alkoholkomponente erhalten werden können.

Durch den Einsatz dieser Oxo-Dicköle sind die erfindungsgemäßen Mischungen von Estern mehrbasiger Carbonsäuren nicht nur besonders preisgünstig zu produzieren sondern es eröffnet sich auch eine sinnvolle Verwertung der ansonsten nur als Neben- und Abfallprodukt geltenden Oxo-Dicköle. Die Verwendung der Oxo-Dicköle bei dem erfindungsgemäßen Herstellungsverfahren stellt eine besonders bevorzugte Ausführungsform desselben dar und auch die so hergestellten erfindungsgemäßen Estergemische stellen eine besonders bevorzugte Ausführungsform der vorliegenden Erfindung dar.

Zur Ausführung der vorliegenden Erfindung eignen sich Oxo-Dicköle, die
0 bis 20 Gew.-%, vorzugsweise 4 bis 15 Gew.-% Oxoalkohole $C_9$ - $C_{15}$
0 bis 40 Gew.-%, vorzugsweise 20 bis 35 Gew.-% 2-Alkylalkohole $C_{18}$ - $C_{30}$
0 bis 10 Gew.-%, vorzugsweise 1 bis 5 Gew.-% Sek. Alkohole $C_{18}$ - $C_{30}$
0 bis 20 Gew.-%, vorzugsweise 3 bis 20 Gew.-% Etheralkohole $C_{18}$ - $C_{30}$
0 bis 25 Gew.-%, vorzugsweise 5 bis 25 Gew.-% Esteralkohole $C_{27}$ - $C_{51}$
0 bis 7,5 Gew.-%, vorzugsweise 0,8 bis 5 Gew.-% 1,2-Diole $C_{10}$ - $C_{16}$
0 bis 8 Gew.-%, vorzugsweise 1 bis 5 Gew.-% Diole $C_{18}$ - $C_{30}$
0 bis 45 Gew.-%, vorzugsweise 10 bis 40 Gew.-% von OH-Gruppen-freie Ether $C_{18}$ - $C_{45}$ und Ester $C_{18}$ - $C_{45}$ umfassen,

und eine OH-Zahl im Bereich von 65 bis 160, vorzugsweise von 90 bis 140 aufweisen.
Zur Auswahl geeigneter Oxo-Dicköle ist es lediglich erforderlich, die zur Verfügung stehenden Produkte daraufhin zu untersuchen, ob sie die oben beschriebene erforderliche Zusammensetzung aufweisen.

Die qualitative Untersuchung von Oxo-Dickölen und die quantitative Bestimmung der darin enthaltenen Haupt-Bestandteile kann in an sich bekannter Weise (Vergl. z.B. EP-A-0 718 351) durch gaschromatographische Trennung mit angekoppelter massenspektrographischer Untersuchung der erhaltenen Fraktionen erfolgen.
Zweckmäßigerweise arbeitet man unter Einsatz von 25 m oder 50 m SE 54 Fused Silica Kapillaren. Abbildungen von so erhaltenen Gaschromatogrammen finden sich auf den Seiten 14 und 15 der oben genannten EP-A-0 718 351. Durch Ausmessen der Peakflächen der Hauptkomponenten und Normierung auf 100% kann die Zusammensetzung der Oxo-Dicköle mit einer für die Ausführung der vorliegenden Erfindung ausreichenden Genauigkeit ermittelt werden.

Speziell - aber keineswegs ausschließlich - geeignet zur Herstellung der erfindungsgemäßen Ester sind die Oxodicköle der Typen "Oxoöl 911", "Oxoöl 13" und "Oxoöl 135".

Die erfindungsgemäßen Estergemische können durch Überführung zumindest eines Teils der darin enthaltenen freien Sulfonsäuregruppen und eventuell noch vorhandener Carbonsäuregruppen in Metallsalze (M in Formel I zumindest teilweise = Metallatom), vorzugsweise in Alkalisalze, insbesondere in Natrium- oder Kaliumsalze, d.h. nach Neutralisation oder Teilneutralisation der Carbonsäuregruppen, in eine wasserlösliche oder wasserdispergierbare Form gebracht und dann als Dispergiermittel bzw. als Emulgatoren zur stabilen Feinverteilung wasserunlöslicher Substanzen in einer wässrigen Phase eingesetzt werden, die sich durch eine sehr gute Wirksamkeit und sehr günstige Gestehungskosten auszeichnen. Die Verwendung der erfindungsgemäßen Estergemische als Emulgatoren ist ebenfalls ein Gegenstand der vorliegenden Erfindung.

Die Eigenschaften der erfindungsgemäßen Gemische von Estern der Formel I können durch Zusatzstoffe variiert werden. Durch solche Variationen kann der Anwendungsbereich der Gemische erweitert werden, das heißt es können neue Anwendungsgebiete erschlossen und bei den weiter unten beschriebenen bevorzugten Verwendungen speziellen Forderungen der Verwender Rechnung getragen werden. Eine Gruppe von Zusatzstoffen, besteht aus Mischungen der strukturell verwandten Monoester der Formeln Ia und Ib

$$(MOOC)_d\text{-}R^1\text{-}CO\text{-}OR^2 \qquad (Ia)$$

$$[(MOOC)_d\text{-}R^1\text{-}CO\text{-}O]_2R^3 \qquad (Ib)$$

worin d die Ziffern 1 oder 2 bedeutet, M für Wasserstoff oder ein Metalläquivalent steht und R1, R2 und R3 die gleichen Bedeutungen haben, wie in der Definition der Verbindungen der Formel I.
Auch bei den Verbindungen der Formeln Ia und Ib betragen - bezogen auf die Summe SUG der Gewichte der in der Mischung enthaltenen von OH-Gruppen freien Ether, von OH-Gruppen freien Ester, Alkanole, Alkandiole und $-OR^2$- und $(-O)_2R^3$-Gruppen, - der Anteil der $-OR^2$-Gruppen bis zu 85 %, der Anteil der $(-O)_2R^3$-Gruppen bis zu 16 %.
Die Verbindungen der Formeln Ia und Ib können im Bedarfsfall den erfindungsgemäßen Estergemischen der Formel I nach deren Herstellung zugesetzt werden, sie können jedoch zweckmäßigerweise auch direkt bei der Herstellung der erfindungsgemäßen Estergemische erzeugt werden, indem man einfach die Veresterungsreaktion in dem Moment abbricht, wo der gewünschte Anteil der Monoester der Formeln Ia und Ib im Reaktionsgemisch vorliegt. Von technischem Interesse können auch Estergemische sein, die einen sehr hohen Anteil der Monoester der Formeln Ia und Ib aufweisen. In diesem Fall wirken die erfindungsgemäßen Ester der Formel I als Modifizierungsmittel um die anwendungstechnischen Eigenschaften der Monoester Ia und Ib speziellen Forderungen anzupassen. Technisch interessante modifizierende Effekte können in der Regel beim Zusatz von ca 5 Gew.-% Monoestern Ia/Ib zu Diestern I beziehungsweise von 5 Gew.-% Diestern I zu Monoestern Ia/Ib beobachtet werden. Bei geringeren Zusätzen ergeben sich entsprechend geringere Modifizierungseffekte.

Eine besonders vorteilhafte Verwendung, die ebenfalls ein Gegenstand dieser Erfindung ist, besteht im Einsatz der erfindungsgemäßen Estergemische als Hilfsmittel bei der Herstellung von Leder. Hierbei können die erfindungsgemäßen Estergemische mit besonderem Vorteil, unter anderem auch wegen ihrer guten Dispergierwirkung und ihrer guten Verträglichkeit mit anderen Hilfsmittel der Lederherstellung, in Kombination mit Lederfettungsmitteln und/oder Lederhydrophobierungsmitteln eingesetzt werden.

Als fettende und/oder hydrophobierende Mittel kommen insbesondere unveresterte Oxo-dicköle in Betracht. Zusätzlich zu diesen oder an deren Stelle können jedoch auch weitere Substanzen, wie z.B. Weißöl, Paraffine, native Öle, Silikone und/oder hydrophil modifizierte Copolymere als fettende und/oder hydrophobierende Mittel eingesetzt werden.

Besonders gute Hydrophobierungseffekte werden erhalten, wenn die erfindungsgemäßen Estergemische mit Silikonen und den unveresterten Oxodickölen und/oder gegebenenfalls weiteren fettenden und/oder hydrophobierenden Substanzen kombiniert werden. Als Silikone, die sich für diesen Einsatz eignen, sind insbesondere bekannte Polysiloxane zu nennen, die an einer linearen oder verzweigten Siloxan-Grundkette über Brückenglieder gebundene Carboxylgruppen

tragen. Diese Verbindungen entsprechen vorzugsweise der Formel IV

$$[\text{Siloxan-Grundkette-}][\text{-BR(-COOH)}_p]_y, \qquad (IV)$$

worin BR ein (p+1)-valentes an ein Si-Atom der Grundkette gebundenes organisches Brückenglied ist, p für eine Zahl von 1 bis 10 steht und y so gewählt wird, daß die Verbindung 0,01 bis 2,0, vorzugsweise 0,02 bis 1,5 meq/g Carboxylgruppen enthält.

Geeignete Brückenbausteine BR entsprechen beispielsweise der Formel (V)

$$\text{-Z-(A-)}_p \qquad (V),$$

worin A ein divalenter aliphatischer geradkettiger oder verzweigter, ein divalenter cyclischer oder bicyclischer, gesättigter oder ungesättigter oder ein divalenter aromatischer Kohlenwasserstoffrest ist, Z eine direkte Bindung, ein Sauerstoffatom oder eine Gruppe der Formel $-NR^4-$, -CO-, oder -CO-O- oder einen (p+1)-valenten organischen Rest der Formel Va

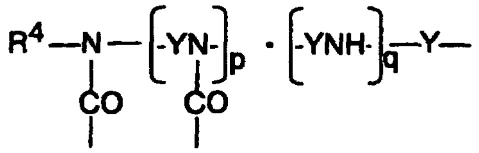

(Va)

bedeutet, wobei p und q unabhängig voneinander für die Zahlen von 0 bis 10 stehen und die Summe p+q ebenfalls im Bereich von 0 bis 10 liegt, und $R^4$ in den genannten Baugruppen Wasserstoff oder $C_1$- bis $C_4$-Alkyl und Y gleiche oder verschiedene geradkettige oder verzweigte Alkandiyl-Reste mit 2 bis 4 C-Atomen bedeuten.

Geeignete Polysiloxane mit linearer Siloxan-Grundkette sind beispielsweise aus der EP-B-0 745 141 bekannt, als Beispiele für Siloxane mit linearer oder verzweigter Grundkette seien die aus der WO-98/21369 bekannten Verbindungen genannt.

Hydrophil modifizierte Copolymere, die sich als Zusätze zu den erfindungsgemäßen Estergemischen eignen und zu einer noch weiteren Verbesserung und Erweiterung des Eigenschaftsspektrums im Hinblick auf die Anwendung als Hilfsmittel bei der Lederherstellung führen, sind solche, die hydrophile Gruppen tragen, insbesondere protische Gruppen, die zur Bildung von Wasserstoffbrücken befähigt sind. Als sehr gut geeignet haben sich Copolymere der Formel VI

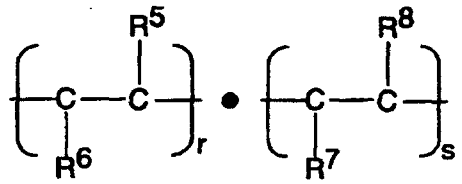

(VI)

erwiesen, worin

| | |
|---|---|
| $R^5$ | Wasserstoff oder Alkyl und $R^6$ Alkyl bedeutet, wobei $R^5$ und $R^6$ gemeinsam im Mittel 8 bis 30, vorzugsweise 10 bis 22, C-Atome aufweisen, |
| $R^6$ | ferner Alkoxycarbonyl mit 2 bis 8, vorzugsweise 3 bis 6, insbesondere 4 C-Atome hat, |
| $R^7$ | eine Carboxyl- oder Amidocarbonylgruppe und |
| $R^8$ | Wasserstoff oder eine Carboxylgruppe bedeutet, |
| r und s | zweckmäßigerweise Zahlenwerte von 5 bis 50 sind, wobei das Verhältnis r:s im Bereich von 1:1 bis 10:1 liegt. |

Besonders vorteilhaft sind Copolymere in denen

$R^5$ Wasserstoff oder eine Alkylgruppe und $R^6$ eine Alkylgruppe bedeutet, die gemeinsam im Mittel 8 bis 30, vorzugsweise 10 bis 22, C-Atome aufweisen, und $R^7$ eine Carboxylgruppe und $R^8$ Wasserstoff oder eine Carboxylgruppe, vorzugsweise eine Carboxylgruppe, bedeutet,

ferner solche Copolymere der Formel V, in denen

$R^5$ und $R^8$ jeweils Wasserstoff, $R^6$ Alkoxycarbonyl mit 2 bis 8, vorzugsweise 3 bis 6, insbesondere 4 C-Atomen und

$R^7$ eine Carboxyl- oder Amidocarbonylgruppe bedeutet.

Copolymere der Formel VI, die sich als Zusätze zu den erfindungsgemäßen Estergemischen eignen, sind im Handel erhältlich und/oder können nach bekannten Verfahren hergestellt werden.

Bei der bevorzugten Verwendung der erfindungsgemäßen Estergemische als Lederhilfsmittel oder als Bestandteil derselben werden diese zweckmäßigerweise in Form von wäßrigen Zubereitungen eingesetzt, die neben den erfindungsgemäßen Estergemischen und gegebenenfalls darin enthaltenen Monoestern der Formeln Ia und Ib noch weitere zur Fettung und/oder Hydrophobierung geeignete Substanzen als Emulsion in Wasser enthalten können. Im Prinzip werden zur Emulgierung dieser Fettungs- und Hydrophobierungsmittel neben den erfindungsgemäßen Estergemischen keine weiteren amphiphilen Komponenten benötigt; sie können aber zugesetzt werden, wenn spezielle Effekte gewünscht werden. Als fettende und/oder hydrophobierende Komponenten kommen für diese Zubereitungen die oben genannten Substanzen, insbesondere unveresterte Oxodicköle in Betracht. Neben oder anstelle von unveresterten Oxodickölen können jedoch auch weitere Substanzen, wie z.B. Weißöl, Paraffine, native Öle die oben beschriebenen Copolymere und/oder Silikone in die Zubereitungen eingearbeitet werden, wobei Zubereitungen, die einen Anteil der Copolymeren und/oder von Silikonen enthalten, besonders hohe Hydrophobierungseffekte ergeben. Zweckmäßigerweise enthalten derartige Zubereitungen, 1 bis 20, vorzugsweise 2 bis 10, insbesondere 2,5 bis 8 Gew.-% der Copolymere und/oder Silikone.

Die besagten wäßrigen Zubereitungen sind ebenfalls ein Gegenstand der vorliegenden Erfindung.

Sie enthalten in der Regel neben Wasser, bezogen auf den nicht flüchtigen Anteil

2 bis 50 Gew.-% der erfindungsgemäßen Estergemische der Formel I und/oder deren Salze,

0 bis 50 Gew.-% Monoestergemische der der Formeln Ia und Ib,

wobei der Gesamtanteil der Verbindungen der Formeln I, Ia und Ib im nicht flüchtigen Anteil 10 bis 50 Gew.% beträgt,

0 bis 20 Gew.-% weitere Emulgiermittel

0 bis 90 Gew.-% nicht verestertes Oxodicköl

0 bis 90 Gew.-% weitere hydrophobierende Substanzen der oben genannten Klassen,

0 bis 10 Gew.-% Hilfsmittel, wie Schaumdämpfungsmittel, Frostschutzmittel, Bakterizide, Fungizide, Metallkomplexbildner, Lagerstabilisatoren, Verdürmungshilfsmittel und dergleichen.

Der Feststoffgehalt der wäßrigen Zubereitungen liegt zweckmäßigerweise bei 20 bis 60 Gew.-%, kann aber, wenn es gewünscht wird, niedriger oder höher eingestellt und damit besonderen Anwendern, Anwendungsbereichen und Anlageneifordernissen angepaßt werden.

Selbstverständlich ist es auch möglich, wasserfreie Zubereitungen der oben angegebenen Zusammensetzung herzustellen und bei Bedarf einzusetzen. Auch diese wasserfreien Zubereitungen sind daher Gegenstand dieser Erfindung.

Die erfindungsgemäßen wäßrigen Zubereitungen stellen relativ niederviskose Flüssigkeiten dar, weisen vergleichsweise niedrige Emulgatorkonzentrationen auf, haben eine sehr gute Lagerstabilität, sind sehr unempfindlich gegen Wasserhärte und Nachgerbstoffe und lassen sich ohne Schwierigkeiten auf die für die Lederbehandlung erforderliche Anwendungskonzentration verdünnen.

Die damit behandelten Leder haben keine schmierige Oberfläche, einen angenehmen Griff, sind gleichmäßig gefärbt und wasserundurchlässig. Die erhaltenen Effekte zeigen eine sehr gute Beständigkeit gegen Wasser, wäßrige Tensidlösungen und chemische Reinigungsmittel.

Auch der Einsatz der erfindungsgemäßen Ester als Hilfsmittel in Lederbehandlungsverfahren ist ein Gegenstand der vorliegenden Erfindung. Für diesen Einsatz werden die Ester zweckmäßigerweise in Form der oben beschriebenen Zubereitungen, vorzugsweise der wäßrigen Zubereitungen, verwendet. In der Regel werden den Lederbehandlungsflotten, bezogen auf das Falzgewicht des Leders (wet blue) 0,5 bis 8, vorzugsweise 1,5 bis 5 Gew.-% der in der erfindungsgemäßen Zubereitungen enthaltenen nichtflüchtigen Anteile zugesetzt. Im Übrigen erfolgt die Lederbehandlung in bekannter Weise.

Die folgenden Ausführungsbeispiele dienen der Veranschaulichung der Erfindung. Die Zusammensetzung des darin eingesetzten Typs von Oxodicköl ist der folgenden Tabelle 2 zu entnehmen:

**Tabelle 2**

| Oxodicköl-Type | "Oxoöl 135" |
|---|---|
| Komponenten | Anteile in Gew.-% |
| Oxoalkohole $C_9$ - $C_{15}$ | 6,1 |
| 2-Alkylalkohole $C_{18}$ - $C_{30}$ | 27,9 |
| Sek. Alkohole $C_{18}$ - $C_{30}$ | 4,1 |
| Etheralkohole $C_{18}$ - $C_{30}$ | 6,7 |

Tabelle fortgesetzt

| | |
|---|---|
| Esteralkohole $C_{27}$ - $C_{45}$ | ca. 17,4 |
| 1,2-Diole $C_{10}$ - $C_{16}$ | 1,0 |
| Diole $C_{18}$ - $C_{30}$ | 2,3 |
| Ether $C_{18}$ - $C_{45}$ (OH-Gruppen-frei) | 5,4 |
| Ester $C_{18}$ - $C_{45}$(OH-Gruppen-frei) | ca. 29,1 |

Die qualitative Untersuchung der Oxoöle 911 und 135 und die quantitative Bestimmung der in Tabelle 2 angegebenen Anteilswerte der Bestandteile erfolgte in an sich bekannter Weise durch gaschromatographische Trennung und angekoppelter massenspektrographischer Untersuchung der erhaltenen Fraktionen. Die Trennung wurde bei Oxoöl 911 unter Einsatz einer 50 m SE 54 Fused Silica Kapillare ausgeführt. Für die Trennung des Oxoöls 135 wurde eine entsprechende 25 m Kapillare verwendet. Die in der Tabelle angegebenen Hauptkomponenten wurden durch Normierung der GC-Peakflächen auf 100% ermittelt.

Das in den Beispielen eingesetzte Oxoöl 135 ist außerdem durch eine OH-Zahl von 107 charakterisiert. Die Bestimmung der OH-Zahl erfolgte in Anlehnung an die Norm DIN-53240 vom Dezember 1971 bzw. DiN-53240 Teil II vom Dezember 1993.

Die Prüfung der Leder auf Wasserdurchlässigkeit erfolgte mit dem Bally-Penetrometer gemäß IUP 10 der Internationalen Union der Leder-Chemiker-Verbärtde.(Vergl. "Das Leder", Bd. 12, Seiten 26-40 (1961))

**Beispiel 1.**

A) Herstellung eines erfindungsgemäßen Esters.

In einem 2000 ml Dreihals-Glaskolben, ausgerüstet mit Rührer, Thermometer, Destillationsaufsatz und Feuchtigkeitsverschluß werden 940 g (1,8 Mol nach OH-Zahl) Oxodicköl Typ 135 vorgelegt und im Ölbad auf 100°C erwärmt. Unter Rühren werden dann 88,2 g (0,9 Mol) Maleinsäureanhydrid eingetragen und die Mischung 7 Stunden bei 100°C gerührt.

Dann werden dem Ansatz 3 g Toluolsulfonsäure zugesetzt, das Ölbad auf 130°C geheizt und der Ansatz in einem leichten Wasserstrahlvakuum (25 bis 50 mbar) unter Abdestillieren des Wassers weitergerührt, bis keine nennenswerten Wassermengen mehr gebildet werden.

Der so erhaltene Ester wird auf 80°C abgekühlt; der Umsatz ist praktisch quantitativ.

In einem 2000 ml Vierhalskolben mit Schnellrührer, Thermometer Entlüftung und Tropftrichter wird eine Lösung von 85 g Natriumdisulfit ($Na_2S_2O_5$) in 400 ml Wasser vorgelegt. (Anstelle dieser Lösung kann auch eine äquivalente Menge einer bei anderen technischen Prozessen anfallende "Bisulfitlauge" eingesetzt werden).

Die Sulfitlösung wird auf 80°C erwärmt und im Verlauf von 2 Stunden das gemäß Abschnitt A erhaltene, 80°C warme Estergemisch zugetropft.

Dann wird der Ansatz bei 80°C 6 Stunden weitergerührt und die erhaltene Mischung durch weiteren Zusatz von Wasser auf einen Estergehalt von 50$\pm$2 Gew.-% verdünnt..

B) Herstellung einer erfindungsgemäßen Zubereitung.

400 g der im Abschnitt A hergestellten 50 gew.-%igen Mischung des Ester/Sulfit-Addukts mit Wasser werden bei 40°C unter Rühren durch vorsichtigen Zusatz von 25 gew.-%iger Natronlauge auf einen pH-Wert von 8 eingestellt. Danach werden 340 g Oxodicköl Typ 135, 225 g Weißöl und 1000 g Wasser eingerührt und die Mischung in üblicher Weise, z.B. mittels eines Spalthomogenisators, in eine Emulsion überführt.

Die dünnflüssige Emulsion enthält 10 Gew.-% des erfindungsgemäßen Esters. Sie hat eine sehr gute Lagerbeständigkeit (Stabilität bei 23°C, 40°C und 50°C mehr als 60 Tage) und läßt sich sehr leicht mit Wasser auf Anwendungskonzentration verdünnen. Sie ist beständig gegenüber Hartwasser bis mindestens 40°dH.

In analoger Weise können auch Oxodicköle der Typen 911 und 13 umgesetzt und in Zubereitungen überführt werden.

**Beispiel 2.**

B) Herstellung eines erfindunizsgemäßen Diester/Monoester-Gemisches

In einem 2000 ml Dreihals-Glaskolben, ausgerüstet mit Rührer, Thermometer, Destillationsaufsatz und Feuchtigkeitsverschluß werden 940 g (1,8 Mol nach OH-Zahl) Oxodicköl Typ 135 vorgelegt und im Ölbad auf 100°C erwärmt. Unter Rühren werden dann 88,2 g (0,9 Mol) Maleinsäureanhydrid eingetragen und die Mischung 7 Stunden bei 100°C gerührt.
Dann werden dem Ansatz 3 g Toluolsulfonsäure zugesetzt, das Ölbad auf 130°C geheizt und der Ansatz in einem leichten Wasserstrahlvakuum (25 bis 50 mbar) unter Abdestillieren des Wassers weitergerührt, bis 12 ml Wasser abdestilliert sind.
Das so erhaltene Estergemisch wird auf 80°C abgekühlt; es enthält, bezogen auf Gesamt-Ester, ca 66 Gew.% Diester und 33 Gew.-% Monoester.
In einem 2000 ml Vierhalskolben mit Schnellrührer, Thermometer Entlüftung und Tropftrichter wird eine Lösung von 85 g Natriumdisulfit ($Na_2S_2O_5$) in 400 ml Wasser vorgelegt. (Anstelle dieser Lösung kann auch eine äquivalente Menge einer bei anderen technischen Prozessen anfallende "Bisulfitlauge" eingesetzt werden).
Die Sulfitlösung wird auf 80°C erwärmt und im Verlauf von 2 Stunden das gemäß Abschnitt A erhaltene, 80°C warme Estergemisch zugetropft.
Dann wird der Ansatz bei 80°C 6 Stunden weitergerührt und die erhaltene Mischung durch weiteren Zusatz von Wasser auf einen Estergehalt von $50 \pm 2$ Gew.-% verdünnt..

B) Herstellung einer erfindungsgemäßen Zubereitung.

Zur Herstellung einer Zubereitung verfährt man analog der Beschreibung in Beispiel 1, Abschnitt B.
Die dünnflüssige Emulsion enthält 10 Gew.-% des erfindungsgemäßen Ester-Gemisches. Sie hat ebenfalls eine sehr gute Lagerbeständigkeit (Stabilität bei 23°C, 40°C und 50°C mehr als 60 Tage) und läßt sich sehr leicht mit Wasser auf Anwendungskonzentration verdünnen. Sie ist beständig gegenüber Hartwasser bis mindestens 40°dH.

**Beispiel 3.**

A) Herstellung eines erfindungsgemäßen Esters.

In einem 1000 ml Dreihals-Glaskolben, ausgerüstet mit Rührer, Thermometer, Rückflußkühler und Feuchtigkeitsverschluß werden 470 g (0,9 Mol nach OH-Zahl) Oxodicköl Typ 135 vorgelegt und im Ölbad auf 100°C erwärmt. Unter Rühren werden dann 102,6 g (0,45 Mol) 4-Sulfo-phthalsäureanhydrid eingetragen und die Mischung 7 Stunden bei 100°C gerührt.
Dann wird das Ölbad auf 130°C geheizt und der Ansatz in einem leichten Wasserstrahlvakuum (25 bis 50 mbar) unter Abdestillieren des Wassers weitergerührt, bis keine nennenswerten Wassermengen mehr gebildet werden.
Der so erhaltene Ester wird auf 40°C abgekühlt; der Umsatz ist praktisch quantitativ.

B) Herstellung einer erfindungsgemäßen Zubereitung.

200 g des im Abschnitt A hergestellten Estergemisches werden mit 200 ml Wasser gut verrührt und die Mischung bei 40°C unter Rühren durch vorsichtigen Zusatz von 25 gew.-%iger Natronlauge auf einen pH-Wert von 8 eingestellt. Danach werden 340 g Oxodicköl Typ 135, 225 g Weißöl und 1000 g Wasser eingerührt und die Mischung in üblicher Weise, z.B. mittels eines Spalthomogenisators, in eine Emulsion überführt.
Die dünnflüssige Emulsion enthält 10 Gew.-% des erfindungsgemäßen Esters. Sie hat eine sehr gute Lagerbeständigkeit (Stabilität bei 23°C, 40°C und 50°C mehr als 60 Tage) und läßt sich sehr leicht mit Wasser auf Anwendungskonzentration verdünnen. Sie ist beständig gegenüber Hartwasser bis mindestens 40°dH.
In analoger Weise können auch Oxodicköle der Typen 911 und 13 umgesetzt und in Zubereitungen überführt werden.
Den in den Beispielen 1 bis 3 hergestellten Zubereitungen können auch zusätzlich oder anstelle des Weißöls bis zu 250g der oben beschriebenen hydrophilen Copolymere, insbesondere $C_{12}/C_{24}$ Olefin-Maleinsäure-Copolymere oder Acrylamid-Butylacrylat-Copolymere und/oder bis zu 100g Silikonöle beispielsweise der Formel VII

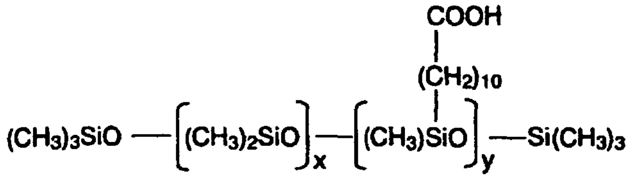

(VII)

worin die Summe von x und y ca. 140 bis 150 beträgt und y einen Wert von ca. 3 hat, zugesetzt werden.

**Eigenschaften erfindungsgemäßer Zubereitungen**

Die gemäß den Beispielen 1 bis 3 erhaltenen Zubereitungen haben, wie oben angegeben, eine sehr gute Lagerbeständigkeit und lassen sich ohne Schwierigkeiten mit Wasser auf Anwendungskonzentration verdünnen.
Selbst eine gemäß Beispiel 4 hergestellte erfindungsgemäße Zubereitung, die nur 5 Gew.-% des erfindungsgemäßen Estergemisches enthielt, weist bei 23°C, 40°C und 60°C eine Lagerstabilität von über 60 Tagen auf
Zum Vergleich wurde eine Zubereitung gemäß Beispiel 1, Abschnitt B, hergestellt, die anstelle des erfindungsgemäßen Estergemisches 10 Gew.% N-Oleoylsarkosin, eines handelsüblichen, sehr gut wirksamen, und daher trotz seines relativ hohen Preises im Hydmphobienmittel-Bereich verbreiteten Emulgators, enthielt. Diese Zubereitung zeigte eine erheblich geringere Lagerstabilität: Bei einer Lagerung bei 23°C trat bereits nach 6 Tagen eine wäßrige Trennung auf (20% der Emulsion sind relativ klar).
Erst eine Zubereitung mit 15 Gew.-% dieses handelsüblichen Emulgators blieb bei einer 23°C-Lagerung mehr als 14 Tage stabil.
Schon allein der Mengenvergleich der zur Stabilisierung der Zubereitungen benötigten Emulgatoren zeigt, daß erst die etwa dreifache Menge des Sarkosin-Emulgators annähernd die Wirksamkeit des erfindungsgemäßen Emulgatorsystems erreicht.
Zieht man darüberhinaus die Wirkungs/Kosten-Relation in Betracht, dann ergibt sich eine mindestens zehnfache Überlegenheit des erfindungsgemäßen Emulgators gegenüber dem guten, handelsüblichen Produkt.

**Anwendungstechnische Beispiele.**

**Beispiel 4.**

Chromgegerbtes Rindleder (wetblue) der Falzstärke 1,8 - 2,0 mm, das auf einen pH-Wert von 5,0 entsäuert worden ist, wurde, bezogen auf Falzgewicht,

| | |
|---|---|
| 30 min. mit | 2 Gew.-% eines handelsüblichen Polymergerbstoffs, danach |
| 60 min. mit | 3 Gew.-% handelsüblichem Mimosaextrak, |
| | 3 Gew.-% eines handelsüblichen Harzgerbstoffs auf Basis von Melaminkondensationsprodukten und |
| | 1 Gew.-% eines handelsüblichen Nachgerbstoffs auf Basis von Phenolkondensadonsprodukten, und danach |
| 60 min. mit | 2 Gew.-%.eines handelsüblichen Lederfarbstoffs im Gerbfaß gewalkt. |

Anschließend wurde, bezogen auf Falzgewicht,
90 min. mit 10 Gew.-% einer Zubereitung, hergestellt gemäß Beispiel 1
gewalkt und die Flotte mit Ameisensäure auf einen pH-Wert von ca. 3,6 bis 3,8 abgesäuert und danach gewaschen.
Abschließend erfolgte für die Dauer von 90 min. eine Mineralsalzfixierung mit 3 Gew.-% eines handelsüblichen Chromgerbstoffs im Gerbfaß.
Dann wurde das Leder gewaschen, mechanisch ausgereckt und getrocknet.
Das erhaltene Leder war weich, geschmeidig, mit angenehmem Griff und gleichmäßiger Färbung.
Die Prüfung mittels Bally-Penetrometer bei 15 % Stauchung erbrachte folgende Ergebnisse:

| | |
|---|---|
| Wasserdurchtritt: | nach 55 min., |
| dynamische Wasseraufnahme: | ca. 32 Gew.-% nach 6 Stunden. |

**Beispiel 5**

Man arbeitet genau wie im Beispiel 4 beschrieben, jedoch wird in die Zubereitung aus Beispiel 1 zusätzlich 80 g des Silicons der Formel VII eingearbeitet.

Die Prüfung mittels Bally-Penetrometer bei 15 % Stauchung erbrachte folgende Ergebnisse:

Kein Wasserdurchtritt nach 24 Stunden,

dynamische Wasseraufnahme: ca. 24 Gew.-% nach 24 Stunden.

## Patentansprüche

**1.** Gemische von Estern zwei- oder dreibasiger Carbonsäuren der Formel I

$$[(MOOC)_{a-b}\text{-}R^1\text{-}(COO\text{-})_b]_m \bullet [\text{-}R^2]_n \bullet [\text{-}R^3\text{-}]_p \bullet [OH]_q \qquad (I)$$

worin a und b jeweils die Ziffern 2 oder 3 bedeuten,wobei b ≤ a ist,
m eine ganze Zahl von 1 bis 6, vorzugsweise von 1 bis 3, ist,
wobei die Zusammenhänge

$$0 \leq n \leq (b-2)m + 2$$

$$(m-1) \leq p \leq (b-1)m + 1$$

bestehen

$$q = (b-2)m + 2 - n$$

ist,
M für Wasserstoff oder ein Metalläquivalent steht und

$R^1$ ein zwei- oder dreiwertiger, gesättigter oder ein- oder zweifach ungesättigter aliphatischer oder cycloaliphatischer Kohlenwasserstoffrest mit 2 bis 6 C-Atomen ist, wobei mindestens 50 Mol% dieser Reste $R^1$ gesättigt oder höchstens einfach ungesättigt sind und eine Sulfonsäuregruppe aufweisen, oder ein zwei- oder dreiwertiger, eine Sulfonsäuregruppe tragender aromatischer Kohlenwasserstoffrest mit 6 C-Atomen ist,
$R^2$ überwiegend verzweigtes, gegebenenfalls durch Alkoxy-, Alkylcarbonyloxy- oder Alkoxycarbonyl substituiertes Alkyl mit 9 bis 51, vorzugsweise 9 bis 45 C-Atomen und
$R^3$ Alkandiyl mit 10 bis 30 C-Atomen bedeutet,

mit von OH-Gruppen freien Ethern und Estern mit 18 bis 45 C-Atomen und gegebenenfalls Alkanolen der Formel $R^2OH$ und Alkandiolen der Formel $R^3(OH)_2$, wobei, - bezogen auf die Summe SUG der Gewichte der in der Mischung enthaltenen von OH-Gruppen freien Ether, von OH-Gruppen freien Ester, Alkanole, Alkandiole und -$OR^2$- und (-O)$_2R^3$-Gruppen, - der Anteil der -$OR^{2-}$Gruppen bis zu 85 %, der Anteil der (-O)$_2R^3$-Gruppen bis zu 16 %, und der Anteil der von OH-Gruppen freien Ether und Ester bis zu 45 % beträgt.

**2.** Gemische von Estern gemäß Anspruch 1 der Formel I

$$[(MOOC)_{a-b}\text{-}R^1\text{-}(COO\text{-})_b]_m \bullet [\text{-}R^2]_n \bullet [\text{-}R^3\text{-}]_p \bullet [OH]q \qquad (I),$$

worin a, b, m, n, p, q und M jeweils die oben angegebenen Bedeutungen haben **dadurch gekennzeichnet, daß**

$R^1$ ein zweiwertiger, gesättigter oder einfach ungesättigter aliphatischer oder cycloaliphatischer Kohlenwasserstoffrest mit 2 bis 6 C-Atomen ist, wobei mindestens 50 Mol% dieser Reste $R^1$ eine Sulfonsäuregruppe aufweisen, oder ein zweiwertiger, eine Sulfonsäuregruppe tragender aromatischer Kohlenwasserstoffrest mit 6 C-Atomen ist.

**3.** Gemische von Estern gemäß den Ansprüchen 1 und 2, **dadurch gekennzeichnet, daß**

$R^1$ ein zweiwertiger, gesättigter oder einfach ungesättigter aliphatischer Kohlenwasserstoffrest mit 2 bis 6, insbesondere 2 bis 4, C-Atomen ist, wobei mindestens 50 Mol%, vorzugsweise mindestens 75 Mol%, insbesondere mindestens 90 Mol% dieser Reste eine Sulfonsäuregruppe aufweisen.

**4.** Gemische von Estern gemäß den Ansprüchen 1 bis 3, mit von OH-Gruppen freien Ethern und Estern mit 18 bis 45 C-Atomen, und gegebenenfalls Alkanolen der Formel $R^2OH$ und Alkandiolen der Formel $R^3(OH)_2$, **dadurch gekennzeichnet, daß**

$R^2$ die Reste $R^{2A}$, $R^{2B}$, $R^{2C}$, $R^{2D}$ und $R^{2E}$ urmaßt, wobei
$R^{2A}$ Alkyl(1)- und Alkyl(2)-Reste mit 9 bis 15 C-Atomen, mit einem mittleren Molgewicht von MA
$R^{2B}$ 2-Alkyl-alkyl(1)-Reste mit 18 bis 30 C-Atomen, mit einem mittleren Molgewicht von MB
$R^{2c}$ x-Alkyl-alkyl(y)-Reste mit 18 bis 30 C-Atomen, mit einem mittleren Molgewicht von MC
$R^{2D}$ Alkoxyalkyl(1)-Reste mit 18 bis 30 C-Atomen, mit einem mittleren Molgewicht von MD
$R^{2E}$ x-Alkylcarbonyloxy-alkyl(y)-Reste und/oder x-Alkoxycarbonyl-alkyl(y)-Reste mit 27 bis 51 C-Atomen mit einem mittleren Molgewicht von ME bedeutet,
$R^3$ die Reste $R^{3F}$ und $R^{3G}$ umfaßt, wobei
$R^{3F}$ Alkandiyl(1,2) und/oder 2-Alkyl-alkandiyl(1,3) mit 10 bis 16 C-Atomen, mit einem mittleren Molgewicht von MF
$R^{3G}$ Alkyl-alkandiyl(1,3) mit 18 bis 30 C-Atomen ist, mit einem mittleren Molgewicht von MG bedeutet,

wobei, - bezogen auf die Summe SUG der Gewichte der in der Mischung enthaltenen von OH-Gruppen freien Ether und Ester, Alkanole, Alkandiole und $-OR^2$- und $(-O)_2R^3$-Gruppen,-
die Gruppen $-OR^{2A}$ einen Anteil von A[%] = 0 bis 20 Gew.-%,
die Gruppen $-OR^{2B}$ einen Anteil von B[%] = 0 bis 40 Gew.-%,
die Gruppen $-OR^{2C}$ einen Anteil von C[%] = 0 bis 10 Gew.-%,
die Gruppen $-OR^{2D}$ einen Anteil von D[%] = 0 bis 20 Gew.-%,
die Gruppen $-OR^{2E}$ einen Anteil von E[%] = 0 bis 25 Gew.-%,
die Gruppen $(-O)_2R^{3F}$- einen Anteil von F[%] = 0 bis 7,5 Gew.-%,
die Gruppen $(-O)_2R^{3G}$- einen Anteil von G[%] = 0 bis 8 Gew.-%,
und die von OH-Gruppen freien Ether und Ester einen Anteil von H[%] = 0 bis 45 Gew.% haben.

**5.** Verfahren zur Herstellung der oben beschriebenen Gemische von Estern der Formel I gemäß Anspruch 1 durch Umsetzung funktioneller Derivate zwei- oder dreibasiger Carbonsäuren mit Alkanolen mittlerer und/oder größerer Kettenlänge, das **dadurch gekennzeichnet ist, daß** man

A) funktionelle Derivate von Di- oder Tricarbonsäuren der Formel III

$$R^1(COX)_a \qquad (III)$$

worin a die Ziffern 2 oder 3 bedeutet,

$R^1$ ein zwei- oder dreiwertiger, gesättigter aliphatischer oder cycloaliphatischer Kohlenwasserstoffrest mit 2 bis 6 C-Atomen ist, der durch eine Sulfonsäuregruppe substituiert ist,
oder ein ein- oder zweifach ungesättigter aliphatischer oder cycloaliphatischer Kohlenwasserstoffrest mit 2 bis 6 C-Atomen ist, der durch eine Sulfonsäuregruppe substituiert sein kann,
oder ein zwei- oder dreiwertiger, eine Sulfonsäuregruppe tragender aromatischer Kohlenwasserstoffrest mit 6 C-Atomen ist,
wobei $R^1$ zumindest entweder eine Sulfonsäuregruppe oder eine olefinische Doppelbindung enthält,
X Hydroxy, Niederalkoxy, oder Halogen bedeuten oder zwei X gemeinsam für Sauerstoff stehen,

bei einer Temperatur von ca. 50 bis 180°C, vorzugsweise von 75 bis 150°C, im Molverhältnis von 1:2 bis 1:5, vorzugsweise in Gegenwart von Veresterungs- oder Umesterungskatalysatoren und/oder gegebenenfalls Säurefängem, mit einer Mischung von
Alkoholen der Formel $R^2OH$, worin
$R^2$ überwiegend verzweigtes, gegebenenfalls durch Alkoxy-, Alkylcarbonyloxy- oder Alkoxycarbonyl substituiertes Alkyl mit 9 bis 51, vorzugsweise 9 bis 45 C-Atomen bedeutet,
Alkandiolen der Formel $(HO)_2R^3$, worin
$R^3$ Alkandiyl mit 10 bis 30 C-Atomen bedeutet,
und von OH-Gruppen freien Ethern und Estern mit 18 bis 45 C-Atomen umsetzt, und,
B) falls für den Herstellungsschritt A) ein von Sulfonsäuregruppen freies, jedoch mindestens eine olefinische Doppelbindung aufweisendes Carbonsäure-Derivat (III) eingesetzt wird, das gemäß Abschnitt A) erhaltene Ester-Gemisch, gegebenenfalls nach zumindest teilweiser Neutralisation saurer Gruppen, in an sich bekannter Weise mit einem wasserlöslichen Sulfit, Hydrogensulfit oder Disulfit unter Addition des Sulfits bzw. Hydrogensulfits an die Doppelbindung der Gruppe $R^1$ unter Bildung der gewünschten Sulfonsäure umsetzt.

6. Verfahren gemäß Anspruch 5, **dadurch gekennzeichnet, daß** als Alkanolkomponente Oxo-Dicköle eingesetzt werden.

7. Wäßrige und wasserfreie Zubereitungen der Estergemische des Anspruchs 1, die gegebenenfalls Monoester der Formeln Ia und Ib

$$(MOOC)_d\text{-}R^1\text{-}CO\text{-}OR^2 \qquad (Ia)$$

$$[(MOOC)_d\text{-}R^1\text{-}CO\text{-}O]_2R^3 \qquad (Ib),$$

worin d die Ziffern 1 oder 2 bedeutet, M für Wasserstoff oder ein Metalläquivalent steht und R1, R2 und R3 die gleichen Bedeutungen haben, wie in der Definition der Verbindungen der Formel I, und wobei - bezogen auf die Summe SUG der Gewichte der in der Mischung enthaltenen von OH-Gruppen freien Ether, von OH-Gruppen freien Ester, Alkanole, Alkandiole und $-OR^2$- und $(-O)_2R^3$-Gruppen, - der Anteil der $-OR^2$-Gruppen bis zu 85 %, der Anteil der $(-O)_2R^3$-Gruppen bis zu 16 % betragen, und zusätzlich zu den Estergemischen fettende und/oder hydrophobierende Substanzen, insbesondere Oxo-dicköle, Weißöl, Paraffine, native Öle, Silikone und/oder hydrophil modifizierte Copolymere enthalten.

8. Wäßrige und wasserfreie Zubereitungen gemäß Anspruch 7, **dadurch gekennzeichnet, daß** sie als Silikone Polysiloxane enthalten, die an einer linearen oder verzweigten Siloxan-Grundkette über Brückenglieder gebundene Carboxylgruppen tragen.

9. Wäßrige und wasserfreie Zubereitungen gemäß Anspruch 7, **dadurch gekennzeichnet, daß** sie als hydrophil modifizierte Copolymere solche enthalten, die hydrophile Gruppen tragen, insbesondere protische Gruppen, die zur Bildung von Wasserstoffbrücken befähigt sind.

10. Die Verwendung der erfindungsgemäßen Estergemische gemäß den Ansprüchen 1 bis 4 und deren Zubereitungen als Hilfsmittel bei der Herstellung von Leder und als Emulgatoren.

## Claims

1. Mixtures of esters of di- or tribasic carboxylic acids of the formula I

$$[(MOOC)_{a-b}\text{-}R^1\text{-}(COO\text{-})_b]_m \bullet [\text{-}R^2]_n \bullet [\text{-}R^3\text{-}]_p \bullet [OH]_q \qquad (I)$$

where a and b are each 2 or 3 subject to the proviso that b is $\leq$ a,
m is an integer from 1 to 6, preferably from 1 to 3, subject to the relationships

$$0 \leq n \leq (b - 2)m + 2$$

$$(m - 1) \leq p \leq (b - 1)m + 1$$

$$q \text{ is } = (b - 2)m + 2 - n,$$

M is hydrogen or a metal equivalent,

$R^1$ is a di- or trivalent, saturated or mono- or diunsaturated aliphatic or cycloaliphatic hydrocarbon radical of 2 to 6 carbon atoms, at least 50 mol% of these $R^1$ radicals being saturated or at most monounsaturated and having a sulfonic acid group, or is a di- or trivalent aromatic hydrocarbon radical of 6 carbon atoms which bears a sulfonic acid group,
$R^2$ is predominantly branched alkyl of 9 to 51, preferably 9 to 45, carbon atoms with or without alkoxy, alkylcarbonyloxy or alkoxycarbonyl substitution, and
$R^3$ is alkanediyl of 10 to 30 carbon atoms,

with OH-free ethers and esters of 18 to 45 carbon atoms and if appropriate alkanols of the formula $R^2OH$ and alkanediols of the formula $R^3(OH)_2$, the fraction of $-OR^2$ groups being up to 85%, the fraction of $(-O)_2R^3$ groups being up to 16% and the fraction of OH-free ethers and esters being up to 45%, based on the sum total SUG of the weights of the OH-free ethers, OH-free esters, alkanols, alkanediols and $-OR^2$ and $(-O)_2{}^{R3}$ groups present in the mixture.

**2.** Mixtures according to claim 1 of the formula I

$$[(MOOC)_{a-b}\text{-}R^1\text{-}(COO\text{-})_b]_m \bullet [\text{-}R^2]_n \bullet [\text{-}R^3\text{-}]_p \bullet [OH]_q \qquad (I)$$

where a, b, m, n, p, q and M are each as defined above, wherein

$R^1$ is a divalent, saturated or monounsaturated aliphatic or cycloaliphatic hydrocarbon radical of 2 to 6 carbon atoms, at least 50 mol% of these $R^1$ radicals having a sulfonic acid group, or is a divalent aromatic hydrocarbon radical of 6 carbon atoms which bears a sulfonic acid group.

**3.** Mixtures according to either of claims 1 and 2, wherein

$R^1$ is a divalent, saturated or monounsaturated, aliphatic hydrocarbon radical of 2 to 6, especially 2 to 4, carbon atoms, at least 50 mol%, preferably at least 75 mol%, especially at least 90 mol%, of these radicals having a sulfonic acid group.

**4.** Mixtures according to any of claims 1 to 3 with OH-free ethers and esters of 18 to 45 carbon atoms and if appropriate alkanols of the formula $R^2OH$ and alkanediols of the formula $R^3(OH)_2$, wherein

$R^2$ comprises $R^{2A}$, $R^{2B}$, $R^{2C}$, $R^{2D}$ and $R^{2E}$, where
$R^{2A}$ denotes 1-alkyl and 2-alkyl radicals of 9 to 15 carbon atoms having an average molecular weight of MA
$R^{2B}$ denotes 2-alkyl-1-alkyl radicals of 18 to 30 carbon atoms having an average molecular weight of MB
$R^{2c}$ denotes x-alkyl-y-alkyl radicals of 18 to 30 carbon atoms having an average molecular weight of MC
$R^{2D}$ denotes 1-alkoxyalkyl radicals of 18 to 30 carbon atoms having an average molecular weight of MD
$R^{2E}$ denotes x-alkylcarbonyloxy-y-alkyl radicals and/or x-alkoxycarbonyl-y-alkyl radicals of 27 to 51 carbon atoms having an average molecular weight of ME,
$R^3$ comprises $R^{3F}$ and $R^{3G}$, where
$R^{3F}$ denotes 1,2-alkanediyl and/or 2-alkyl-1,3-alkanediyl of 10 to 16 carbon atoms having an average molecular weight of MF
$R^{3G}$ denotes 1,3-alkylalkanediyl of 18 to 30 carbon atoms having an average molecular weight of MG,

the $-OR^{2A}$ groups having a fraction of A[%] = 0 to 20% by weight
the $-OR^{2B}$ groups having a fraction of B[%] = 0 to 40% by weight
the $-OR^{2C}$ groups having a fraction of C[%] = 0 to 10% by weight
the $-OR^{2D}$ groups having a fraction of D[%] = 0 to 20% by weight
the $-OR^{2E}$ groups having a fraction of E[%] = 0 to 25% by weight
the $(-O)_2R^{3F}$ groups having a fraction of F[%] = 0 to 7.5% by weight

the $-(O)_2R^{3G}$ groups having a fraction of G[%] = 0 to 8% by weight
and the OH-free ethers and esters have a fraction of H[%] = 0 to 45% by weight,
based on the sum total SUG of the weights of the OH-free ethers and esters, alkanols, alkanediols and $-OR^2$ and $(-O)_2R^3$ groups present in the mixture.

**5.** A process for preparing the above-described mixtures of esters of the formula I according to claim 1 by reacting functional derivatives of di- or tribasic carboxylic acids with alkanols of medium and/or relatively large chain length, which comprises

A) reacting functional derivatives of di- or tricarboxylic acids of the formula III

$$R^1(COX)_a \qquad (III)$$

where a is 2 or 3,

$R^1$ is a di- or trivalent, saturated aliphatic or cycloaliphatic hydrocarbon radical of 2 to 6 carbon atoms which is substituted by a sulfonic acid group, or a mono- or diunsaturated aliphatic or cycloaliphatic hydrocarbon radical of 2 to 6 carbon atoms which may be substituted by a sulfonic acid group,
or is a di- or trivalent aromatic hydrocarbon radical of 6 carbon atoms which bears a sulfonic acid group, subject to the proviso that $R^1$ comprises at least either a sulfonic acid group or an olefinic double bond,
X is hydroxyl, lower alkoxy or halogen or two Xs combine to form oxygen,

at from about 50 to 180°C, preferably from 75 to 150°C in a molecular ratio from 1:2 to 1:5, preferably in the presence of esterification or transesterification catalysts and/or, if appropriate, acid traps, with a mixture of alcohols of the formula $R^2OH$, where
$R^2$ is predominantly branched alkyl of 9 to 51, preferably 9 to 45 carbon atoms with or without alkoxy, alkylcarbonyloxy or alkoxycarbonyl substitution,
alkanediols of the formula $(HO)_2R^3$, where
$R^3$ is alkanediyl of 10 to 30 carbon atoms, and OH-free ethers and esters of 18 to 45 carbon atoms, and
B) when the preparation step A) is carried out using a carboxylic acid derivative (III) which is free of sulfonic acid groups but has at least one olefinic double bond, reacting the ester mixture obtained according to section A), if appropriate after at least partial neutralization of acidic groups, in a conventional manner with a watersoluble sulfite, bisulfite or disulfite to add the sulfite or bisulfite to the double bond of the $R^1$ group to form the desired sulfonic acid.

**6.** The process according to claim 6, wherein the alkanol component used is oxo thick oils.

**7.** Aqueous and nonaqueous formulations of the ester mixtures of claim 1, comprising as well as said mixtures fatliquoring and/or hydrophobicizing substances, especially oxo thick oils, white oil, paraffins, native oils, silicones and/or hydrophilic modified copolymers and if appropriate monoesters of the formulae Ia and Ib

$$(MOOC)_d\text{-}R^1\text{-}CO\text{-}OR^2 \qquad (Ia),$$

$$[(MOOC)_d\text{-}R^1\text{-}CO\text{-}O]_2R^3 \qquad (Ib),$$

where d is 1 or 2, M is hydrogen or a metal equivalent and $R^1$, $R^2$ and $R^3$ have the same meaning as in the definition of the compounds of the formula I and the fraction of $-OR^2$ groups being up to 85%, the fraction of $(-O)_2R^3$ groups being up to 16% based on the sum total SUG of the weights of the OH-free ethers, OH-free esters, alkanols, alkanediols and $-OR^2$ and $(-O)_2R^3$ groups present in the mixture.

**8.** Aqueous and nonaqueous formulations according to claim 7, comprising, as silicones, polysiloxanes bearing carboxyl groups attached via bridge members to a linear or branched siloxane backbone.

**9.** Aqueous and nonaqueous formulations according to claim 7, wherein said hydrophilic modified copolymers bear hydrophilic groups, especially protic groups, capable of forming hydrogen bonds.

**10.** The use of the invention's ester mixtures according to claims 1 to 4 and of their formulations as assistants in leather making and as emulsifiers.

**Revendications**

**1.** Mélanges d'esters d'acides carboxyliques dibasiques ou tribasiques de la formule I :

$$[(MOOC)_{a-b}\text{-}R^1\text{- }(COO\text{-})_b]_m \cdot [\text{-}R^2]_n \cdot [\text{-}R^3\text{-}]_p \cdot [OH]q \qquad (I)$$

dans laquelle a et b représentent chacun les chiffres 2 ou 3, b étant ≤ a,
m est un nombre entier de 1 à 6, de préférence de 1 à 3,
òu les relations sont

$$0 \leq n \leq (b-2)m + 2$$

$$(m-1) \leq p \leq (b-1)m+1$$

et

$$q = (b-2)m+2-n,$$

M représente de l'hydrogène ou un équivalent métallique, et
$R^1$ est un radical d'hydrocarbure aliphatique ou cycloaliphatique bivalent ou trivalent, saturé ou insaturé à une ou deux reprises et comportant 2 à 6 atomes de C, au moins 50 % molaires de ces radicaux $R^1$ étant saturés ou au maximum insaturés une seule fois et présentant un groupe acide sulfonique, ou un radical d'hydrocarbure aromatique divalent ou trivalent, portant un groupe acide sulfonique et comportant 6 atomes de C,
$R^2$ représente un radical alkyle, d'une manière prépondérante ramifié, qui est éventuellement substitué par un groupe alcoxy, alkylcarbonyloxy ou alcoxycarbonyle et qui comporte 9 à 51 atomes de C, de préférence 9 à 45, et
$R^3$ représente un radical alcanediyle comportant 10 à 30 atomes de C,

avec des éthers et esters exempts de groupes OH comportant 18 à 45 atomes de C et éventuellement des alcanols de la formule $R^2OH$ et des alcanediols de la formule $R^3(OH)_2$, la fraction des groupes $-OR^2$, par rapport à la somme SUG des poids des éthers exempts de groupes OH, esters exempts de groupes OH, alcanols, alcanediols et groupes $-OR^2$ et $(-O)_2R^3$ contenus dans le mélange, allant jusqu'à 85 %, la fraction des groupes $(-O)_2R^3$ allant jusqu'à 16 % et la fraction des éthers et esters exempts de groupes OH allant jusqu'à 45 %.

**2.** Mélanges d'esters suivant la revendication 1 de la formule I :

$$[(MOOC)_{a-b}\text{-}R^1\text{-}(COO\text{-})_b]_m \cdot [\text{-}R^2]_n \cdot [\text{-}R^3\text{-}]_p \cdot [OH]q \qquad (I)$$

dans laquelle a, b, m, n, p, q et M ont chacun les significations indiquées ci-dessus, **caractérisés en ce que**

$R^1$ est un radical d'hydrocarbure aliphatique ou cycloaliphatique divalent, saturé ou insaturé à une reprise et comportant 2 à 6 atomes de C, au moins 50 % molaires de ces radicaux $R^1$ présentant un groupe acide sulfonique, ou un radical d'hydrocarbure aromatique divalent, portant un groupe acide sulfonique et comportant 6 atomes de C.

**3.** Mélanges d'esters suivant les revendications 1 et 2, **caractérisés en ce que**

$R^1$ est un radical d'hydrocarbure aliphatique divalent, saturé ou insaturé à une reprise et comportant 2 à 6 atomes de C, en particulier 2 à 4, au moins 50 % molaires, de préférence au moins 75 % molaires, en particulier au moins 90 % molaires, de ces radicaux présentant un groupe acide sulfonique.

**4.** Mélanges d'esters suivant les revendications 1 à 3, avec des éthers et esters exempts de groupes OH comportant 18 à 45 atomes de C, et éventuellement des alcanols de la formule $R^2OH$ et des alcanediols de la formule $R^3(OH)_2$, **caractérisés en ce que**

$R^2$ comporte les radicaux $R^{2A}$, $R^{2B}$, $R^{2C}$, $R^{2D}$ et $R^{2E}$, où
$R^{2A}$ représente des radicaux alkyle(1) et alkyle(2) comportant 9 à 15 atomes de C, avec un poids moléculaire moyen de MA,
$R^{2B}$ représente des radicaux 2-alkyl-alkyle(1) comportant 18 à 30 atomes de C, avec un poids moléculaire moyen de MB,
$R^{2C}$ représente des radicaux x-alkyl-alkyle(y) comportant 18 à 30 atomes de C, avec un poids moléculaire moyen de MC,
$R^{2D}$ représente des radicaux alcoxyalkyle(1) comportant 18 à 30 atomes de C, avec un poids moléculaire moyen de MD,
$R^{2E}$ représente des radicaux x-alkylcarbonyloxyalkyle(y) et/ou x-alcoxycarbonyl-alkyl(y) comportant 27 à 51 atomes de C, avec un poids moléculaire moyen de ME,
$R^3$ comporte les radicaux $R^{3F}$ et $R^{3G}$, où
$R^{3F}$ représente des radicaux alcanediyle(1,2) et/ou 2-alkyl-alcanediyle(1,3) comportant 10 à 16 atomes de C, avec un poids moléculaire moyen de MF,
$R^{3G}$ représente des radicaux alkyl-alcanediyle(1,3) comportant 18 à 30 atomes de C, avec un poids moléculaire moyen de MG,

et où que, par rapport à la somme SUG des poids des éthers et esters exempts de groupes OH, alcanols, alcanediols et groupes $-OR^2$ et $(-O)_2R^3$,
les groupes $-OR^{2A}$ présentent une fraction de A[%] = 0 à 20 % en poids,
les groupes $-OR^{2B}$ présentent une fraction de B[%] = 0 à 40 % en poids,
les groupes $-OR^{2C}$ présentent une fraction de C[%] = 0 à 10 % en poids,
les groupes $-OR^{2D}$ présentent une fraction de D[%] = 0 à 20 % en poids,
les groupes $-OR^{2E}$ présentent une fraction de E[%] = 0 à 25 % en poids,
les groupes $(-O)_2R^{3F}$ présentent une fraction de F[%] = 0 à 7,5 % en poids,
les groupes $(-O)_2R^{3G}$ présentent une fraction de G[%] = 0 à 8 % en poids,
et les éthers et esters exempts de groupes OH présentent une fraction de H[%] = 0 à 45 % en poids.

**5.** Procédé de préparation des mélanges décrits ci-dessus d'esters de la formule I suivant la revendication 1, par réaction de dérivés fonctionnels d'acides carboxyliques dibasiques ou tribasiques avec des alcanols d'une longueur de chaîne moyenne et/ou grande, **caractérisé en ce que**

A) on fait réagir des dérivés fonctionnels d'acides dicarboxyliques ou tricarboxyliques de la formule III :

$$R^1(COX)_a \qquad (III)$$

dans laquelle a représente les chiffres 2 ou 3,

$R^1$ est un radical d'hydrocarbure aliphatique ou cycloaliphatique divalent ou trivalent, saturé, qui comprend 2 à 6 atomes de C et est substitué par un groupe acide sulfonique,
ou un radical d'hydrocarbure aliphatique ou cycloaliphatique insaturé à une ou deux reprises qui comporte 2 à 6 atomes de C et peut être substitué par un groupe acide sulfonique,
ou un radical d'hydrocarbure aromatique divalent ou trivalent, qui porte un groupe acide sulfonique et comporte 6 atomes de C,
$R^1$ contenant au moins un groupe acide sulfonique ou une double liaison oléfinique,
X représente un groupe hydroxy, alcoxy inférieur ou de l'halogène ou deux X représentent

ensemble de l'oxygène,
à une température d'environ 50 à 180°C, de préférence de 75 à 150°C, dans un rapport molaire de 1/2 à 1/5, de préférence en présence de catalyseurs d'estérification ou de transestérification et/ou éventuellement de fixateurs d'acide,
avec un mélange
d'alcools de la formule $R^2OH$, où
$R^2$représente un radical alkyle d'une manière prépondérante ramifié, qui est éventuellement substitué par un groupe alcoxy, alkylcarbonyloxy ou alcoxycarbonyle et qui comporte 9 à 51 atomes de C, de préférence 9 à 45,
d'alcanediols de la formule $(HO)_2R^3$, où
$R^3$ représente un radical alcanediyle comportant 10 à 30 atomes de C,
et d'éthers et esters exempts de groupes OH qui comportent 18 à 45 atomes de C, et

B) dans le cas où on met en oeuvre pour l'étape de préparation A) un dérivé d'acide carboxylique (III) exempt de groupes acide sulfonique, mais présentant au moins une double liaison oléfinique, on fait réagir le mélange d'esters obtenu selon la section A), éventuellement après neutralisation au moins partielle de groupes acides, avec, d'une manière connue en soi, un sulfite, hydrogénosulfite ou disulfite soluble dans l'eau, avec addition du sulfite ou hydrogénosulfite à la double liaison du groupe $R^1$ avec formation de l'acide sulfonique souhaité.

**6.** Procédé suivant la revendication 5, **caractérisé en ce que**, comme composants alcanol, on met en oeuvre des huiles épaisses oxo.

**7.** Compositions aqueuses et anhydres des mélanges d'esters de la revendication 1, qui contiennent éventuellement des monoesters des formules Ia et Ib :

$$(MOOC)_d\text{-}R^1\text{-}CO\text{-}OR^2 \qquad (Ia)$$

$$[(MOOC)_d\text{-}R^1\text{-}CO\text{-}O]_2R^3 \qquad (Ib)$$

dans lesquelles d représente les chiffres 1 ou 2, M représente de l'hydrogène ou un équivalent métallique et $R^1$, $R^2$ et $R^3$ ont les mêmes significations que dans la définition des composés de la formule I, la fraction des groupes $-OR^2$, par rapport à la somme SUG des poids des éthers exempts de groupes OH, esters exempts de groupes OH, alcanols, alcanediols et groupes $-OR^2$ et $(-O)_2R^3$ contenus dans le mélange, allant jusqu'à 85 %, la fraction des groupes $(-O)_2R^3$ allant jusqu'à 16 %, et,
en supplément des mélanges d'esters, des substances graissantes et/ou rendant hydrophobes, en particulier des huiles épaisses oxo, de l'huile blanche, des paraffines, des huiles naturelles, des silicones et/ou des copolymères modifiés de manière hydrophile.

**8.** Compositions aqueuses et anhydres suivant la revendication 7, **caractérisées en ce qu'**elles contiennent, comme silicones, des polysiloxanes qui portent sur une chaîne de base de siloxane linéaire ou ramifiée des groupes carboxyle fixés par des éléments de pont.

**9.** Compositions aqueuses et anhydres suivant la revendication 7, **caractérisées en ce qu'**elles contiennent, comme copolymères modifiés de manière hydrophile, ceux qui portent des groupes hydrophiles, en particulier des groupes protiques, qui sont capables de former des ponts hydrogène.

**10.** Utilisation des mélanges d'esters suivant l'invention selon les revendications 1 à 4 et de leurs compositions, comme adjuvants pour la préparation de cuir et comme agents émulsionnants.